# FASCICULE DE BREVET EUROPEEN

(11) **EP 2 931 266 B1**
(45) Date de publication et mention de la délivrance du brevet: **05.02.2020**
(21) Numéro de dépôt: 13815459.6
(22) Date de dépôt: 17.12.2013
(51) Int. Cl.: A61K 31/155, A61K 31/495, A61K 31/496, A61P 3/10, G01N 33/50

(54) **COMPOSITION ET KIT COMPRENANT DES DÉRIVÉS DE PIPÉRAZINE ET DE LA METFORMINE, LEUR UTILISATION DANS LE TRAITEMENT DU DIABÈTE**
ZUSAMMENSETZUNG UND KIT MIT PIPERAZINDERIVATEN UND METFORMIN SOWIE VERWENDUNG DAVON BEI DER BEHANDLUNG VON DIABETES
COMPOSITION AND KIT COMPRISING PIPERAZINE DERIVATIVES AND METFORMIN, AND USE THEREOF IN THE TREATMENT OF DIABETES

(30) Priorité: 17.12.2012 FR 1262136
(43) Date de publication de la demande: 21.10.2015
(73) Titulaire: Metabolys, 69372 Lyon Cedex 08 (FR)
(72) Inventeur: MOINET, Gérard, 91400 Orsay (FR); BAVEREL, Gabriel, 69450 Saint Cyr au Mont d'Or (FR)
(74) Mandataire: Lavoix
(86) Numéro de dépôt international: PCT/EP2013/076987
(87) Numéro de publication internationale: WO 2014/095929

(56) Documents cités:
- WO-A1-2012/175707
- WO-A1-2012/175715
- WO-A2-2011/039367
- FR-A1- 2 781 797

## Description

La présente invention concerne des compositions et kit comprenant en association au moins un dérivé de pipérazine ou ses sels pharmaceutiquement acceptables et de la metformine ou ses sels pharmaceutiquement acceptables et leurs utilisations notamment dans le traitement de pathologies associées au syndrome d'insulinorésistance (ou syndrome X), notamment dans le traitement du diabète de type (II).

On observe depuis quelques années une augmentation importante des cas de diabète dans le monde. En 2011, on comptait environ 366 millions de diabétiques dans le monde et les prévisions pour 2030 sont de l'ordre de plus de 550 millions de diabétiques. Le diabète de type (I) (destruction des cellules produisant de l'insuline) est principalement traité par l'injection d'insuline. Le diabète de type (II), qui est le plus répandu (90% des cas de diabète), se caractérise par une résistance des tissus face à l'insuline et nécessite un traitement particulier.

De nombreux composés ont été proposés pour le traitement du diabète, notamment du diabète de type (II). On connaît notamment de EP2781797 des dérivés de pipérazine. On connaît également de WO02/100341 des dérivés phénylés, et de WO2011/039367 une combinaison de la metformine avec un dérivé de pipéridine.

Actuellement, dans environ 40% des cas, le traitement n'est pas efficace et le seuil de glycémie à jeun souhaité de 1,26 g/litre de sang n'est pas atteint.

De plus, plus de 40% des patients traités présentent un taux d'hémoglobine glycosilée (HbA1c) supérieur à 7%, notamment après plusieurs années de traitement.

Il est donc nécessaire de proposer de nouvelles associations permettant un traitement plus efficace des pathologies associées au syndrome d'insulinorésistance et limitant les phénomènes de résistance aux agents actifs.

Un objectif de la présente invention est donc de fournir des compositions et associations efficaces dans le traitement des pathologies associées au syndrome d'insulinorésistance. Un autre objectif encore de la présente invention est de proposer des compositions et associations permettant notamment d'inhiber la néoglucogenèse.

Un autre objectif encore de la présente invention est de fournir un moyen de traitement de pathologies associées au syndrome d'insulinorésistance, notamment diabète de type (II).

D'autres objectifs encore apparaîtront à la lecture de la description de l'invention qui suit.

Ces objectifs sont remplis par la présente invention qui propose une composition comprenant en association de la metformine ou un sel de metformine et au moins un composé de formule (I) ses énantiomères, diastéréoisomères et ses sels pharmaceutiquement acceptables 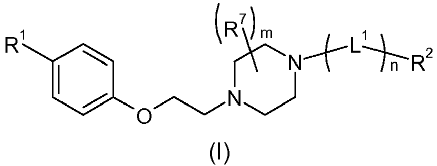 dans laquelle
▪ R¹ représente :
   - un groupe -C(O)CR³R⁴CR⁵R⁶C(O)OH ;
   - un groupe -(CH₂)₄C(O)OH ;
▪ m représente 0 ;
▪ n représente 0 ;
▪ R² représente :
   - un groupe carbocycle en 5, 6 ou 7 membres, saturé, partiellement insaturé ou aromatique, non substitué ou substitué par un ou plusieurs substituants, identiques ou différents, notamment choisi parmi :
      - un groupe alkoxy en C₁ à C₆, de préférence en C₁ à C₃, linéaire ou ramifié, par exemple méthoxy, éthoxy, propoxy, iso-propoxy, butoxy, ter-butoxy ;
      - un atome d'halogène, par exemple fluor, chlore, brome ;
      - un groupe alkyle en C₁ à C₅, de préférence en C₁ à C₄, linéaire ou ramifié, par exemple méthyle, éthyle, propyle, butyle, iso-propyle, ter-butyle ; ou
      - un groupe alkyle en C₁ à C₅, de préférence en C₁ à C₄, linéaire ou ramifié, substitué notamment par un ou plusieurs atomes d'halogène, par exemple trifluorométhyl ;
   - un groupe hétérocycle en 5, 6 ou 7 membres, saturé, partiellement insaturé ou aromatique pouvant comprendre 1, 2 ou 3 hétéroatomes, identiques ou différents, notamment choisis parmi l'azote, l'oxygène ou le soufre, non substitué ou substitué par un ou plusieurs substituants, identiques ou différents, notamment choisi parmi :
      - un groupe alkoxy en C₁ à C₆, de préférence en C₁ à C₃, linéaire ou ramifié, par exemple méthoxy, éthoxy, propoxy, iso-propoxy, butoxy, ter-butoxy ;
      - un atome d'halogène, par exemple fluor, chlore, brome ;
      - un groupe alkyle en C₁ à C₅, de préférence en C₁ à C₄, linéaire ou ramifié, par exemple méthyle, éthyle, propyle, butyle, iso-propyle, ter-butyle; ou
      - un groupe alkyle en Ci à C5, de préférence en Ci à C4, linéaire ou ramifié, substitué notamment par un ou plusieurs atomes d'halogène, par exemple trifluorométhyl.
▪ R³, R⁴, R⁵ et R⁶, identiques ou différents, représentent :
   - un atome d'hydrogène ;
   - un groupe alkyle, linéaire ou ramifié, en C₁ à C₅, de préférence en C₁ à C₄.

De façon préférée, dans le composé de formule (I), R² représente :
- un groupe carbocycle aromatique en 6 membres, non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué notamment par un ou plusieurs atomes d'halogène, par exemple trifluorométhyl ;
- un groupe hétérocycle aromatique en 5 ou 6 membres, comprenant 1, 2 ou 3 hétéroatomes, identiques ou différents, notamment choisis parmi l'azote, le soufre et l'oxygène, non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué notamment par un ou plusieurs atomes d'halogène, par exemple trifluorométhyl.

De façon préférée, dans le composé de formule (I), R² représente :
- un phényl, non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué notamment par un ou plusieurs atomes d'halogène, par exemple trifluorométhyl ;
      de préférence le ou les substituants sont en position ortho ou para sur le phényl ;
- un hétéroaryl monocyclique choisi parmi 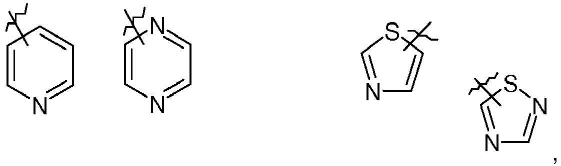 non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué notamment par un ou plusieurs atomes d'halogène, par exemple trifluorométhyl.

De façon préférée, dans le composé de formule (I), R² représente :
- un groupe carbocycle aromatique en 6 membres, de préférence phényle non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué notamment par un ou plusieurs atomes d'halogène, par exemple trifluorométhyl ;
- un groupe hétérocycle aromatique en 5 ou 6 membres comprenant 1, 2 ou 3 hétéroatomes, identiques ou différents, notamment choisis parmi l'azote, le soufre et l'oxygène, de préférence pyridine, non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué notamment par un ou plusieurs atomes d'halogène, par exemple trifluorométhyl.

De préférence dans le composé de formule (I), R² représente :
- un groupe phényle non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
- un groupe pyridine.

De préférence, dans le composé de formule (I), R³, R⁴, R⁵ et R⁶, identiques ou différents, représentent :
- un atome d'hydrogène ;
- un groupe alkyle, linéaire ou ramifié, en C₁ à C₅, de préférence en C₁ à C₄, par exemple méthyle, éthyle, propyle, butyle, iso-propyle, ter-butyle.

De préférence, dans le composé de formule (I), R³, R⁴ représentent chacun un atome d'hydrogène et R⁵ et R⁶, identiques ou différents représentent :
- un atome d'hydrogène ;
- un groupe alkyle, linéaire ou ramifié, en C₁ à C₅, de préférence en C₁ à C₄, par exemple méthyle, éthyle, propyle, butyle, iso-propyle, ter-butyle.

De préférence, dans le composé de formule (I) R³, R⁴, R⁵ et R⁶ représentent chacun un atome d'hydrogène.

Dans un mode de réalisation particulier dans les composés de formule (I), R¹ représente un groupe -C(O)CR³R⁴CR⁵R⁶C(O)OH, R², R³, R⁴, R⁵, R⁶ ayant les définitions précitées.

De préférence dans ce mode de réalisation R³, R⁴, R⁵ et R⁶ représentent chacun un atome d'hydrogène.

De préférence dans ce mode de réalisation R² représente :
- un groupe phényle non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ; ou
   - un atome d'halogène, par exemple fluor, chlore, brome.

De préférence dans ce mode de réalisation, R³, R⁴, R⁵ et R⁶ représentent chacun un atome d'hydrogène et R² représente :
- un groupe phényle non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy.

Dans un mode de réalisation particulier dans les composés de formule (I), R¹ représente un groupe -(CH₂)₄C(O)OH, R² ayant les définitions précitées.

De préférence dans ce mode de réalisation R² représente :
- un groupe phényle non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
- un groupe pyridine.

De préférence dans ce mode de réalisation, R² représente :
- un groupe phényle non substitué ou substitué par un ou plusieurs substituants, identiques ou différents, notamment choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
- un groupe pyridine.

Toutes les caractéristiques générales et préférées des groupes R1, R2, R3, R4, R5, R6, peuvent être combinées ensemble.

De préférence, le composé de formule (I) est choisi parmi :

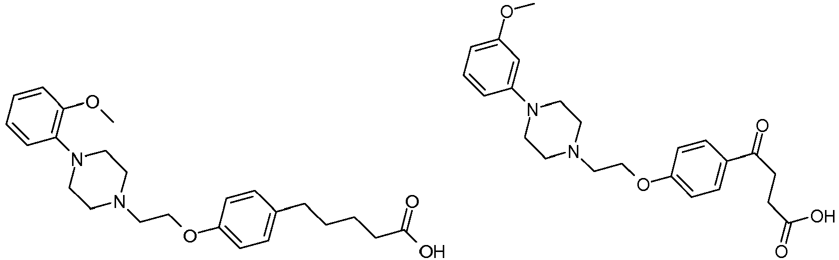

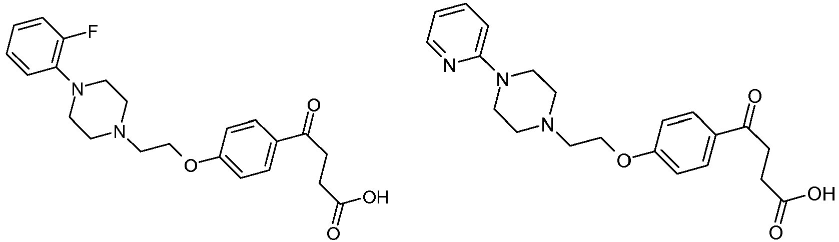

L'invention se rapporte également aux compositions comprenant des solvates des composés de formules (I).

Les composés de formules (I) possèdent une fonction carboxylique et peuvent être salifiés. Ils peuvent alors se présenter sous la forme de sels d'addition avec des bases organiques ou minérales. Les sels d'addition avec des bases sont par exemple des sels pharmaceutiquement acceptables tels que les sels de sodium, les sels de potassium, les sels de calcium, lesquels sont obtenus en utilisant des hydroxydes de métaux alcalins et alcalino-terreux correspondants comme bases. Comme autre type de sels d'addition avec des bases pharmaceutiquement acceptables, on peut citer les sels avec des amines et notamment la glucamine, le N-méthylglucamine, le N,N-diméthylglucamine, l'éthanolamine, la morpholine, la N-méthylmorpholine ou la lysine.

Les composés de formules (I) peuvent également être salifiés avec des acides minéraux ou organiques et de préférence des acides pharmaceutiquement acceptables tels que les acides chlorhydrique, phosphorique, fumarique, citrique, oxalique, sulfurique, ascorbique, tartrique, maléique, mandélique, méthanesulfonique, lactobionique, gluconique, glucarique, succinique, sulfonique ou hydroxypropanesulfonique.

La metformine peut éventuellement être sous la forme de l'un de ses sels pharmaceutiquement acceptables. On peut notamment citer les chlrohydrate, acétate, benzoate, citrate, fumarate, embonate, chlorophénoxyacétate, glycolate, palmoate, aspartate, méthanesulfonate, maléate, parachlorophénoxyisobutyrate, formate, lactate, succinate, sulfate, tartrate, cyclohexanecarboxylate hexanoate, octanoate, décanoate, hexadecanoate, octodecanoate, benzènesulfonate, trimethoxybenzoate, paratoluènesulfonate, adamantanecarboxylate glycoxylate, glutamate, pyrrolidonecarboxylate, naphtalène sulfonate, glucose-1-phosphate, nitrate, sulfite, dithionate, phosphate, de préférence, chlorhydrate, fumarate, embonate, chlorophénoxyacétate.

Les sels de metformine sont obtenus de façon connue par l'homme du métier, notamment par réaction entre la metformine et les acides correspondants aux sels susmentionnés.

Ces compositions peuvent également comprendre un véhicule et/ou excipient pharmaceutiquement acceptable.

Les compositions pharmaceutiques peuvent être sous toutes formes connues par l'homme du métier, notamment sous des formes destinées à l'administration par voie parentérale, orale, rectale, permuqueuse ou percutanée, de préférence par voie orale.

Les compositions selon l'invention seront présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions de ou suspensions, pour l'usage percutané, dans un solvant polaire ou l'usage permuqueux.

Les excipients qui conviennent pour de telles administrations sont les dérivés de la cellulose ou la cellulose microcristalline, les carbonates alcalino-terreux, le phosphate de magnésium, les amidons, les amidons modifiés, le lactose pour des formes solides.

Pour l'usage rectal, le beurre de cacao ou les stéarates de polyéthylène glycol sont les excipients préférés.

Pour l'usage parentéral, l'eau, les solutés aqueux, le sérum physiologique, les solutés isotoniques sont les véhicules les plus commodément utilisés.

L'invention concerne également l'utilisation d'une composition selon l'invention pour la préparation d'un médicament.

L'invention concerne également la composition selon l'invention pour son utilisation en tant que médicament.

L'invention concerne également les compositions de l'invention destinées à une utilisation pour le traitement de pathologies associées au syndrome d'insulinorésistance (syndrome X). Ces pathologies sont notamment mises en évidence dans la publication de Haffner et al. (Diabetes, 1992, 41(6), 715-722).

Les associations de la metformine avec d'autres composés anti-diabétiques aboutissent à une saturation de la réponse et les activités de la metformine et des composés anti-diabétiques ne s'additionnent pas toujours ou pas toujours de manière significative.

De façon tout à fait surprenante et avantageuse, l'association de la metformine avec les composés de formule (I) aboutit à un cumul des activités séparées de la metformine et des composés de formule (I).

La présente invention concerne en particulier les compositions pharmaceutiques de l'invention pour leur utilisation pour le traitement de pathologies associées au syndrome d'insulinorésistance (syndrome X).

La présente invention divulgue en particulier une méthode de traitement des pathologies associées au syndrome d'insulinorésistance comprenant l'administration d'une quantité efficace d'une composition pharmaceutique de l'invention à un patient qui en a besoin.

La présente invention concerne en particulier les compositions pharmaceutiques de l'invention pour la préparation de médicaments pour le traitement de pathologies associées au syndrome d'insulinorésistance.

De préférence, parmi les pathologies associées au syndrome d'insulinorésistance on peut citer le diabète, notamment le diabète de type (II).

De préférence, les compositions de l'invention permettent également l'inhibition de la néoglucogenèse.

La présente invention concerne aussi une composition selon l'invention ou kit comprenant au moins un composé de formule (I) ou un de ses sels et de la metformine ou un sel de metformine pour une administration simultanée, séparée ou séquentielle à un patient qui en a besoin. Pour une administration simultanée, le composé de formule (I) et la metformine peuvent être en mélange dans la même préparation les contenant avec un véhicule ou excipient pharmaceutiquement acceptable. Ils peuvent aussi être conditionnés dans des préparations séparées chacun avec un excipient ou véhicule pharmaceutiquement acceptable, aptes à être mélangés notamment extemporanément. En cas d'administration séparée ou séquentielle, chacun des principes actifs est conditionné dans une préparation propre contenant un véhicule ou excipient pharmaceutiquement acceptable.

De préférence, la composition ou le kit de l'invention comprend de la metformine ou un de ses sels pharmaceutiquement acceptables et un composé choisi parmi : 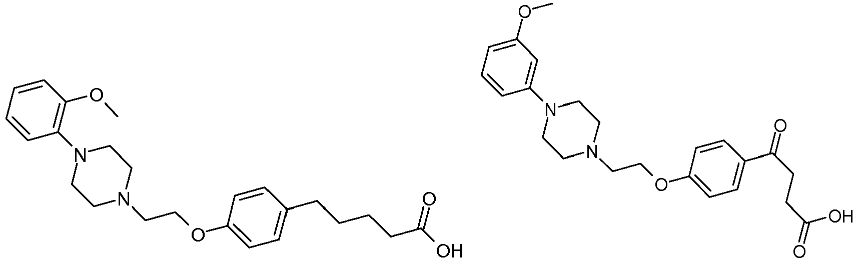 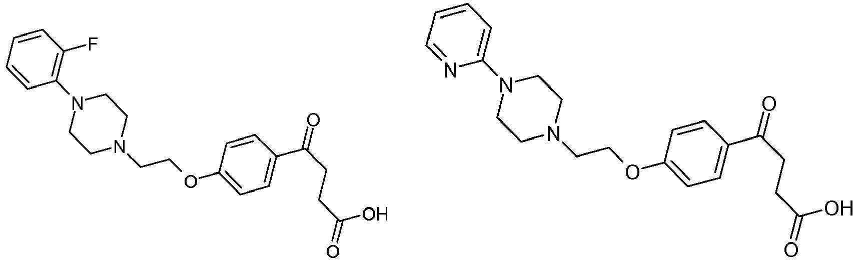 ou un de leurs sels pharmaceutiquement acceptables.

Dans un mode de réalisation, la composition ou le kit de l'invention comprend de la metformine ou un de ses sels pharmaceutiquement acceptables et un composé de formule 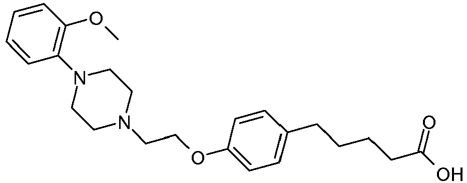 ou un de ses sels pharmaceutiquement acceptables.

De préférence, la composition ou le kit de l'invention comprend de la metformine ou un de ses sels pharmaceutiquement acceptables et un composé choisi parmi : 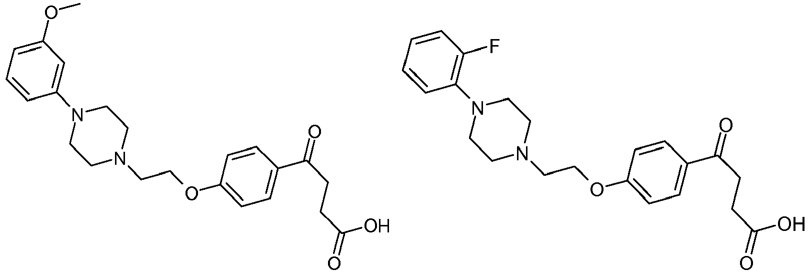 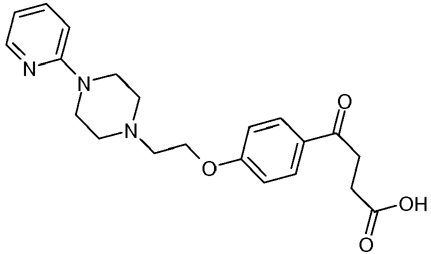 ou un de leurs sels pharmaceutiquement acceptables.

La posologie peut varier dans des limites importantes en fonction de l'indication thérapeutique, et de la voie d'administration, ainsi que de l'âge et du poids du sujet.

L'identification du patient qui a besoin du traitement ci-dessus indiqué est définie par l'homme du métier. On entend par patient un être humain ou un animal. Un médecin ou un vétérinaire peut identifier, par l'intermédiaire de tests cliniques, d'examen physique, de tests ou diagnostics biologiques et par l'historique familial et/ou médical, les sujets qui ont besoin d'un tel traitement.

On entend par quantité suffisante, une quantité de composé selon la présente invention efficace pour prévenir ou traiter des conditions pathologiques. La quantité suffisante peut être déterminée par l'homme du métier, par l'intermédiaire de technique conventionnelle et par l'observation des résultats obtenus dans des circonstances analogues. Pour déterminer la quantité suffisante différents facteurs doivent être pris en compte par l'homme du métier, notamment et sans y être limité : le sujet, sa taille, son âge, son état de santé général, la maladie impliquée et son degré de sévérité ; la réponse du sujet, le type de composé, le mode d'administration, la biodisponibilité de la composition administrée, le dosage, l'utilisation concomitante d'autres concomitante d'autres médicaments, etc. De préférence, dans les compositions de l'invention, le ratio metformine/composé de formule (I) est tel que la metformine est administrée dans une quantité de 200 mg/jour à 3 g/jour et le composé selon l'invention est administré de 2,5 mg/jour à 500 mg/jour, de préférence en une ou deux prise(s).

De préférence, et sauf indications contraires, dans les composés de formule (I) selon l'invention les carbocycles comprennent 5 ou 6 membres, sont saturés, partiellement insaturés ou aromatiques, substitués ou non substitués, de préférence ils comprennent 6 membres et sont aromatiques, par exemple phényle.

De préférence, et sauf indications contraires, dans les composés de formule (I) selon l'invention les hétérocarbocycles comprennent 5 ou 6 membres, et peuvent comprendre 1, 2 ou 3 hétéroatomes, identiques ou différents, notamment choisis parmi l'azote, l'oxygène ou le soufre, ils sont substitués ou non substitués, saturés, partiellement insaturés ou aromatiques, de préférence aromatiques. Ils sont par exemple choisis parmi

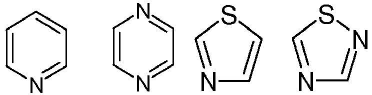

Les composés de formule (I) peuvent être obtenus par un procédé (P1) comprenant:
(a) la protection de la fonction acide (portée par R¹) d'un composé de formule (II) 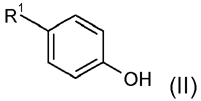 dans laquelle R¹ représente -C(O)CR³R⁴CR⁵R⁶C(O)OH ou -(CH₂)₄C(O)OH;
(b) la réaction du composé obtenu à l'étape (a) avec un composé de formule R⁹-(CH₂)₂-R⁸ dans laquelle R⁸ et R⁹, identiques ou différents représentent un groupe partant, de préférence R⁹ est meilleur nucléofuge que R⁸ ;
(c) la réaction du composé obtenu à l'étape (c) avec un composé de formule (III) 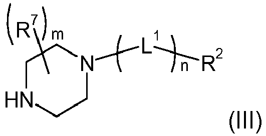 dans laquelle L¹, n, m, R² et R⁷ ont les définitions données pour la formule (I) ;
(d) déprotection du composé obtenu à l'étape (c).

Les produits de départ sont commerciaux ou peuvent être facilement préparés par l'homme du métier sur la base de ses connaissances générales en chimie organique.

L'étape (a), correspondant à la protection de la fonction acide, peut se faire de toute manière connue par l'homme du métier, pourvue que la protection soit sélective de la fonction acide par rapport à la fonction alcool. Parmi les groupes protecteurs de la fonction carboxylique, ceux généralement décrits dans Protective Groups in Organic Synthesis, Greene T.W. et Wuts P.G.M, ed. John Wiley and Sons, 1991 et dans Protective Groups, Kocienski P.J., 1994, Georg Thieme Verlag, peuvent convenir. A titre d'exemple on peut envisager la protection de la fonction carboxylique sous la forme d'ester (alkyle en C₁-C₆, par exemple méthyle). Par exemple l'étape (a) peut se faire par réaction du composé de formule (I) avec le méthanol en présence d'un acide, notamment l'acide sulfurique.

L'étape (b) est de préférence mise en oeuvre dans un solvant polaire aprotique tel que l'acétonitrile, le diméthylformamide (DMF), l'acétone, le diméthylsulfoxide et les hydrocarbures halogénés tels que le dichlorométhane ou le dichloroéthane, à une température adaptée, notamment comprise entre 15 et 80°C, de préférence entre 15 et 35°C. Un solvant préféré est notamment l'acétonitrile. De façon avantageuse, cette étape a lieu en présence d'une base telle que le carbonate de potassium. L'homme du métier sait qu'un groupe partant est d'autant plus labile que l'espèce anionique correspondante est stable. Ainsi, R⁹ est plus nucléofuge que R⁸ correspond au fait que R⁹⁻ est plus stable que R⁸⁻. Les groupes partants R⁸ et R⁹, identiques ou différents, sont choisis parmi les atomes d'halogène, de préférence chlore et brome ; les groupes (C₆-C₁₀)arylsulfonyloxy, le groupe aryle étant éventuellement substitué par un ou plusieurs groupes alkyle en C₁ à C₆ ; les groupes (C₁-C₆)alkylsulfonyloxy dans lequel le groupe alkyle est éventuellement substitué par un ou plusieurs atomes d'halogène. De préférence R⁸ représente le chlore et R⁹ représente le brome.

L'étape (c) est une étape de substitution nucléophile pour laquelle les conditions opératoires pourront être facilement déterminées par l'homme du métier. Avantageusement, la réaction est mise en oeuvre dans un solvant polaire aprotique en présence d'une base. Des exemples de solvant appropriés sont l'acétonitrile, le diméthylformamide, l'acétone, le diméthylsulfoxide et les hydrocarbures halogénés tels que le dichlorométhane ou le dichloroéthane. A titre de base on peut citer les carbonates de métal alcalin ou alcalino-terreux, par exemple le carbonate de potassium. De façon avantageuse, la réaction de l'étape (c) est conduite à une température de 50 à 120°C, par exemple au reflux du solvant en présence d'un iodure de métal alcalin tel que l'iodure de potassium. La quantité d'iodure alcalin peut être variable et dépend essentiellement de la nature des réactifs, de la nature du solvant et de la température réactionnelle. La réaction est généralement stœchiométrique, il est néanmoins possible de travailler avec un léger excès de l'un ou de l'autre des réactifs.

L'étape de déprotection (d) peut être toute déprotection connue de l'homme du métier et compatible avec la protection mise en oeuvre à l'étape (a) permettant de récupérer la fonction acide. Par exemple, il peut s'agir d'une saponification en milieu basique, acide ou catalytique (Pd/C).

Les composés de formule (I) peuvent également être obtenus par un second procédé (P2) comprenant :
(i) la réaction d'un composé de formule (III) avec un composé de formule R⁹-(CH₂)₂-R⁸, dans laquelle R⁸ et R⁹ sont tels que définis dans le procédé (P1) ;
(ii) la protection de la fonction acide d'un composé de formule (II) ;
(iii) la réaction entre le composé obtenu à l'étape (i) et le composé obtenu à l'étape (ii) ;
(iv) la déprotection du composé obtenu à l'étape (iii).

Les produits de départ sont commerciaux ou peuvent être facilement préparés par l'homme du métier sur la base de ses connaissances générales en chimie organique.

L'étape (i) est de préférence mise en œuvre dans un solvant polaire aprotique tel que l'acétonitrile, le diméthylformamide (DMF), l'acétone, le diméthylsulfoxide et les hydrocarbures halogénés tels que le dichlorométhane ou le dichloroéthane, à une température adaptée, notamment comprise entre 15 et 80°C, de préférence entre 15 et 35°C. Un solvant préféré est notamment l'acétonitrile. De façon avantageuse cette étape a lieu en présence d'une base telle que le carbonate de potassium.

L'étape (ii), correspondant à la protection de la fonction acide, peut se faire de toute manière connue par l'homme du métier, pourvue que la protection soit sélective de la fonction acide par rapport à la fonction alcool. Parmi les groupes protecteurs de la fonction carboxylique, ceux généralement décrits dans Protective Groups in Organic Synthesis, Greene T.W. et Wuts P.G.M, ed. John Wiley and Sons, 1991 et dans Protective Groups, Kocienski P.J., 1994, Georg Thieme Verlag, peuvent convenir. A titre d'exemple on peut envisager la protection de la fonction carboxylique sous la forme d'ester (alkyle en C₁-C₆, par exemple méthyle). Par exemple l'étape (a) peut se faire par réaction du composé de formule (I) avec le méthanol en présence d'un acide, notamment l'acide sulfurique.

L'étape (iii) est une étape de substitution nucléophile pour laquelle les conditions opératoires pourront être facilement déterminées par l'homme du métier. Avantageusement, la réaction est mise en oeuvre dans un solvant polaire aprotique en présence d'une base. Des exemples de solvant appropriés sont l'acétonitrile, le diméthylformamide, l'acétone, le diméthylsulfoxide et les hydrocarbures halogénés tels que le dichlorométhane ou le dichloroéthane. A titre de base on peut citer les carbonates de métal alcalin ou alcalino-terreux, par exemple le carbonate de potassium. De façon avantageuse, la réaction de l'étape (c) est conduite à une température de 50 à 120°C, par exemple au reflux du solvant en présence d'un iodure de métal alcalin tel que l'iodure de potassium. La quantité d'iodure alcalin peut être variable et dépend essentiellement de la nature des réactifs, de la nature du solvant et de la température réactionnelle. La réaction est généralement stœchiométrique, il est néanmoins possible de travailler avec un léger excès de l'un ou de l'autre des réactifs.

L'étape de déprotection (iv) peut être toute déprotection connue de l'homme du métier et compatible avec la protection mise en oeuvre à l'étape (ii) permettant de récupérer la fonction acide. Par exemple, il peut s'agir d'une saponification en milieu basique, acide ou catalytique (Pd/C).

De façon avantageuse, les composés de formule (I) selon l'invention agissent concomitamment sur 3 organes cibles :
- le foie par inhibition de la gluconéogenèse (ou néoglucogenèse) hépatique ;
- le muscle squelettique par stimulation de sa consommation de glucose ; et
- le pancréas par stimulation de la sécrétion d'insuline par les cellules bêta.
Aussi, les composés de formule (I) ont le potentiel de corriger les 3 déficits majeurs observés chez des patients souffrant du diabète de type 2.

Le mécanisme d'action des composés de formule (I) est différent de celui de la metformine ce qui permet d'avoir un effet de l'association composé de formule (I) + metformine plus important que l'effet de la metformine seule.

Sur la base de ces informations et des méthodes qui vont être maintenant décrites, l'homme du métier est à même, sans aucune difficulté, de confirmer l'effet additif de n'importe quel composé de formule (I) ou de sélectionner, plus largement, toute molécule pouvant être associées à la metformine.

La présente invention divulgue également une méthode de sélection d'un composé pour le traitement de pathologies associées au syndrome d'insulinorésistance ayant un effet complémentaire à celui de la metformine comprenant les étapes de :
a) disposer d'animaux normaux à jeun ;
b) mesurer la glycémie de ces animaux ;
c) administrer à une partie des animaux le composé à tester, à une deuxième partie des animaux la metformine et à la dernière partie des animaux une association de metformine et du composé à tester ;
d) administrer aux animaux une charge de glucose, notamment de 2 à 4 g/kg de poids corporel de l'animal ;
e) mesurer la glycémie à différentes heures ;
f) déterminer si le composé a un effet additif à l'effet de la metformine sur la baisse de la glycémie, notamment comparer l'aire sous la courbe de glycémie obtenue pour chacun des composés administrés et déterminer si le composé testé en association à la metformine a permis de diminuer l'aire sous la courbe de glycémie par rapport à la metformine administrée seule.

La méthode comprend une étape g) de sélection du composé lorsque celui-ci répond positivement à l'étape f).

La méthode peut également comprendre les étapes b), d) et e) réalisées sur des lots d'animaux témoins auxquels aucun composé n'est administré.

De préférence, les animaux sont des souris normales à jeun, de préférence des souris Swiss, par exemple des souris Swiss de 8 semaines à jeun, notamment depuis 17h.

De préférence, l'administration des composés se fait par voie orale.

De préférence, la charge en glucose se fait par voie orale.

Typiquement, la glycémie est mesurée à l'aide d'un glucomètre et de bandelette de mesure de glycémie.

De préférence, la quantité de composé à tester utilisée est comprise entre 1 et 200 mg/kg de poids corporel de l'animal.

De préférence, la quantité de metformine utilisée est comprise entre 300 mg/kg et 1000 mg/kg de poids corporel de l'animal.

De préférence, l'association metformine + composé à tester comprend de 300 à 1000 mg/kg de poids corporel de l'animal de metformine et de 1 à 200 mg/kg de poids corporel de l'animal de composé à tester.

Selon une modalité, le composé à tester est un composé de formule (I) ou non.

La méthode de sélection divulguée par la présente invention peut également comprendre les étapes préalables de :
1) déterminer la toxicité du composé à tester ;
2) disposer de cellules hépatiques d'animaux à jeun ;
3) incuber ces cellules avec du lactate ou de la glutamine ;
4) ajouter le composé à tester dans le milieu d'incubation ;
5) réaliser un dosage enzymatique de la consommation de lactate ou de glutamine et/ou la production de glucose et/ou un dosage du niveau cellulaire en ATP;
6) déterminer les composés non ou peu toxiques permettant de stimuler l'oxydation complète du lactate ou de la glutamine et/ou de diminuer la concentration cellulaire en ATP ; et/ou
7) disposer d'animaux présentant un diabète de type 2 ;
8) administrer à ces animaux le composé à tester ;
9) administrer aux animaux une charge de glucose notamment de 1 à 3 g/kg de poids corporel de l'animal ;
10) évaluer de la consommation périphérique de glucose par le muscle squelettique et/ou la concentration d'insuline circulante ;
11) déterminer les composés non ou peu toxiques permettant d'augmenter la consommation de glucose dans le muscle et/ou d'augmenter la concentration d'insuline circulante.

La méthode peut également comprendre les étapes 3) et 5) et/ou les étapes 9) et 10) réalisées sur des lots d'animaux témoins auxquels aucun composé n'est administré. De préférence, l'administration des composés se fait par voie orale.

De préférence, la charge en glucose se fait par voie orale.

De préférence, les cellules de l'étape 1) sont des cellules hépatiques de rats à jeun, de préférence à jeun depuis 48h.

De préférence, l'incubation se fait en présence de 0,5 à 5 mM de lactate ou de glutamine.

De préférence, le composé à tester est introduit dans une quantité de 0,5 à 2 mM.

Le dosage enzymatique de la consommation de lactate ou de glutamine, de la production de glucose et le dosage du niveau cellulaire en ATP est réalisé par des méthodes bien connues de l'homme du métier et notamment décrites dans Bergmeyer 1974.

De préférence, le lactate mis en oeuvre est du 2-¹³C-lactate ce qui permet de mieux suivre son oxydation complète notamment par spectroscopie du carbone 13.

La méthode divulguée l'invention comprend alors soit la mise en oeuvre des étapes 1) à 6) puis la mise en oeuvre des étapes a) à f) sur les composés répondant positivement à l'étape 6) soit la mise en œuvre des étapes 7) à 11) puis la mise en œuvre des étapes a) à f) sur les composés répondant positivement à l'étape 11), ou encore la mise en œuvre des étapes 1) à 11) puis la mise en œuvre des étapes a) à f) sur les composés répondant positivement aux étapes 6) et 11).

La présente invention divulgue également une méthode de sélection de composés non ou peu toxiques permettant de stimuler l'oxydation complète du lactate ou de la glutamine et/ou de diminuer la concentration cellulaire en ATP comprenant les étapes suivantes
1) déterminer la toxicité du composé à tester ;
2) disposer de cellules hépatiques d'animaux à jeun ;
3) incuber ces cellules avec du lactate ou de la glutamine ;
4) ajouter le composé à tester dans le milieu d'incubation ;
5) réaliser un dosage enzymatique de la consommation de lactate ou de glutamine et/ou de la production de glucose et/ou un dosage du niveau cellulaire en ATP ;
6) déterminer les composés non ou peu toxiques permettant de stimuler l'oxydation complète du lactate ou de la glutamine et/ou de diminuer la concentration cellulaire en ATP

La méthode peut également comprendre les étapes 3) et 5) réalisées sur des lots d'animaux témoins auxquels aucun composé n'est administré.

La méthode comprend une étape 7) de sélection du composé lorsque celui-ci répond positivement à l'étape 6).

La présente invention divulgue également une méthode de sélection de composés non ou peu toxiques permettant d'augmenter la consommation de glucose dans le muscle et/ou d'augmenter la concentration d'insuline circulante comprenant les étapes de :
1) déterminer la toxicité du composé à tester ;
2) disposer d'animaux présentant un diabète de type 2 ;
3) administrer à ces animaux le composé à tester ;
4) administrer aux animaux une charge de glucose notamment de 1 à 3 g/kg de poids corporel de l'animal ;
5) évaluer la consommation périphérique de glucose par le muscle squelettique et/ou la concentration d'insuline circulante ;
6) déterminer les composés non ou peu toxiques permettant d'augmenter la consommation de glucose dans le muscle et/ou d'augmenter la concentration d'insuline circulante.

La méthode peut également comprendre les étapes 4) et 5) réalisées sur des lots d'animaux témoins auxquels aucun composé n'est administré.

La méthode comprend une étape 7) de sélection du composé lorsque celui-ci répond positivement à l'étape 6).

La présente invention divulgue également une méthode de sélection de composés non ou peu toxiques permettant de stimuler l'oxydation complète du lactate ou de la glutamine, de diminuer la concentration cellulaire en ATP d'augmenter la consommation de glucose dans le muscle et d'augmenter la concentration d'insuline circulante comprenant les étapes suivantes :
1) déterminer la toxicité du composé à tester ;
2) disposer de cellules hépatiques d'animaux à jeun ;
3) incuber ces cellules avec du lactate ou de la glutamine ;
4) ajouter le composé à tester dans le milieu de culture ;
5) réaliser un dosage enzymatique de la consommation de lactate ou de glutamine et un dosage du niveau cellulaire en ATP;
6) déterminer les composés non ou peu toxiques permettant de stimuler l'oxydation complète du lactate ou de la glutamine sans augmenter la concentration cellulaire en ATP ; et
7) disposer d'animaux présentant un diabète de type 2 ;
8) administrer à ces animaux le composé à tester ;
9) administrer aux animaux une charge de glucose notamment de 1 à 3 g/kg de poids corporel de l'animal ;
10) évaluation de la consommation périphérique de glucose par le muscle squelettique et la concentration d'insuline circulante ;
11) déterminer les composés non ou peu toxiques permettant d'augmenter la consommation de glucose dans le muscle et d'augmenter la concentration d'insuline circulante.

La méthode peut également comprendre les étapes 3), 5), 9) et 10) réalisées sur des lots d'animaux témoins auxquels aucun composé n'est administré.

La méthode comprend une étape 12) de sélection du composé lorsque celui-ci répond positivement à l'étape 11).

La présente invention divulgue aussi une méthode de production d'une composition comprenant en association de la metformine ou un sel de metformine et au moins un composé ayant un effet additif sur l'effet de la metformine, par exemple un composé de formule (I), ses énantiomères, diastéréoisomères et ses sels pharmaceutiquement acceptables, dans lequel associé à la metformine un composé ayant répondu positivement à la méthode de sélection qui vient d'être décrite, suivant ses différentes configurations, ou bien l'on soumet le composé à cette méthode de sélection, et si le composé s'est révélé répondre positivement au test, on le retient pour la composition. Cette méthode de production peut comprendre l'association du composé et de la metformine dans une même composition ou dans deux compositions séparées mais à administrer au même patient, par exemples associées dans un kit.

La présente invention va maintenant être décrite à l'aide d'exemples non limitatifs.

### Exemple 1. Schéma général de préparation des composés pour lesquels R¹ représente C(O)CR³R⁴CR⁵R⁶C(O)OH

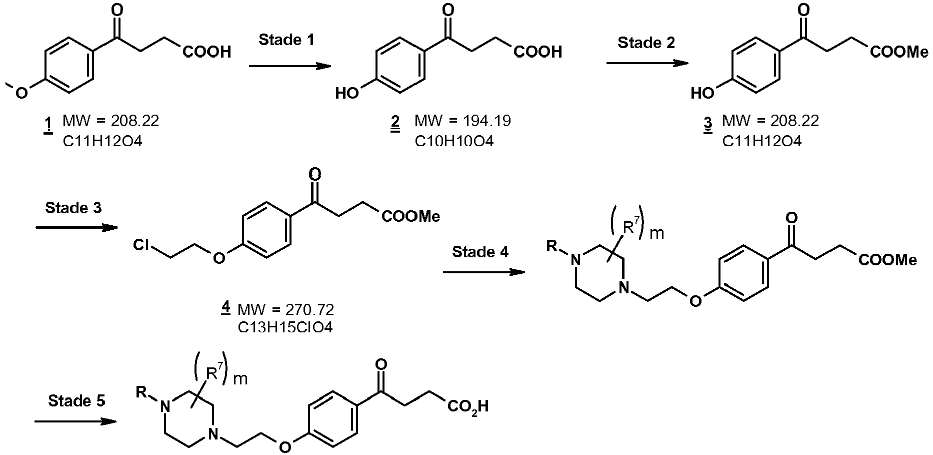

La première étape est une étape de déméthylation pouvant être réalisé par réaction de HBr en milieu acide acétique.

La seconde étape est une réaction d'estérification pouvant être réalisée par réaction avec le méthanol en présence d'acide sulfurique.

Les étapes 3 et 4 sont des étapes de substitution nucléophile.

L'étape 5 peut être réalisée notamment par hydrolyse acide ou saponification.

### Etape 1 : Déméthylation

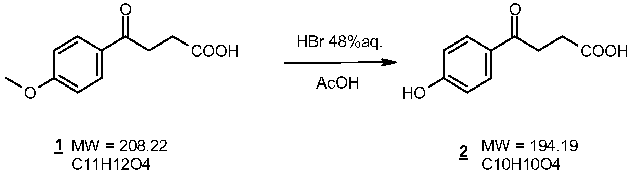

Equipement : Ballon de 1L équipé d'une agitation magnétique et d'un réfrigérant - Bain d'huile.

Le 4-(4-methoxyphenyl)-4-oxobutanone **1** (40g) est mis en solution dans l'acide acétique (360ml) et l'acide bromhydrique à 48% aqueux (120ml). Le milieu réactionnel est chauffé à 130°C pour la nuit.

L'avancement de la réaction est contrôlé par CCM (éluant : Heptane/Acétate d'éthyle : 1/1). Après une nuit d'agitation dans ces conditions, on observe la disparition de l'éther de départ **1** au profit d'un produit plus polaire.

Le milieu réactionnel est concentré à sec sous pression réduite. Le résidu obtenu est repris par de l'eau et la phase aqueuse est extraite trois fois par de l'acétate d'éthyle. Une fois rassemblées, les phases organiques sont lavées une fois par une solution de chlorure de sodium saturée, séchées sur sulfate de magnésium, filtrées puis concentrées sous pression réduite pour fournir un solide (38g).

L'analyse RMN-1H du brut réactionnel révèle la présence du phénol **2** attendu en mélange avec près de 6% d'éther de départ.

Rendement quantitatif.

### Etape 2 : Estérification

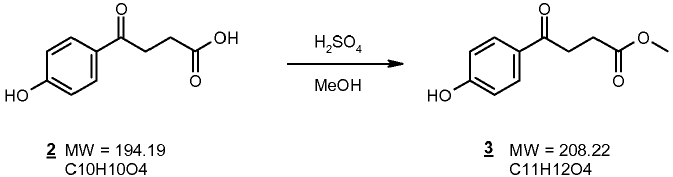

Equipement : Tricol de 1L équipé d'une agitation magnétique, d'un réfrigérant et placé sous balayage d'azote - Bain d'huile.

Le dérivé 4-(4-hydroxyphényl)-4-oxobutanoique acide **2** (48g) est mis en solution dans le méthanol (480ml) avant de couler lentement l'acide sulfurique (48ml). La solution ainsi obtenue est chauffée à 70°C pour la nuit.

L'avancement de la réaction est contrôlé par CCM (éluant : Heptane/Acétate d'éthyle : 1/1). Après une nuit d'agitation dans ces conditions, on observe la disparition de l'acide de départ **2** au profit d'un produit moins polaire.

Le milieu réactionnel est concentré à sec sous pression réduite. Le résidu obtenu est repris par de l'eau (500ml) et par du dichlorométhane (500ml). Le milieu hétérogène est neutralisé, avec précaution, avec une solution saturée d'hydrogénocarbonate de sodium (pH 7-8). La phase aqueuse est ensuite extraite trois fois avec de l'acétate d'éthyle. Une fois rassemblées, les phases organiques sont lavées une fois par une solution de chlorure de sodium saturée, séchées sur sulfate de magnésium, filtrées puis concentrées sous pression réduite pour fournir un solide (49.6g).

L'analyse RMN-1H du brut réactionnel révèle la présence de l'ester 3 attendu en mélange avec moins de 3% du composé 4-(4-méthoxyphényl)-4-oxobutanoate de méthyle.

Rendement 96%.

### Etape 3 : Substitution nucléophile

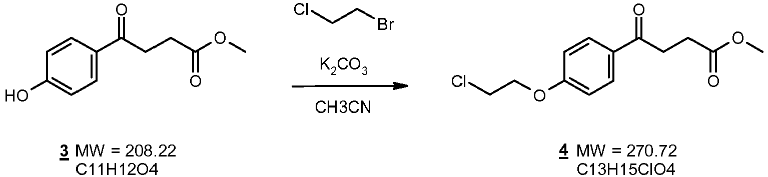

Equipement : Tricol de 1L équipé d'une agitation magnétique, d'un réfrigérant et placé sous balayage d'azote - Bain d'huile.

Le 4-(4-hydroxyphényl)-4-oxobutanoate de méthyle **3** (49,6g) est mis en solution dans l'acétonitrile (400ml). Le carbonate de potassium (98,77g) est ajouté à la solution. Le milieu réactionnel est chauffé à 50°C avant de couler une solution de 1-bromo-2-chloroéthane (102,5g) dans l'acétonitrile (170ml). Le milieu réactionnel est chauffé à 80°C pour la nuit.

L'avancement de la réaction est contrôlé par CCM (éluant : Heptane/Acétate d'éthyle : 7/3). Après 24H d'agitation dans ces conditions, on observe la disparition de composé **3** au profit d'un produit moins polaire.

Après retour à température ambiante, le milieu réactionnel est filtré pour éliminer le carbonate de potassium. Le carbonate de potassium est rincé avec de l'acétonitrile puis le filtrat est concentré à sec sous pression réduite. Le résidu obtenu est repris par de l'eau (500ml) et la phase aqueuse est extraite deux fois par de l'acétate d'éthyle (600ml). Les phases organiques sont rassemblées, lavées une fois avec une solution d'hydroxyde de sodium 1M (400ml), séchées sur sulfate de magnésium, filtrées puis concentrées sous pression réduite pour fournir un solide beige (61,44g).

L'analyse RMN-1H du brut réactionnel révèle la présence du dérivé chloré **4** attendu.

Rendement 95%.

### Etape 4 : Substitution nucléophile

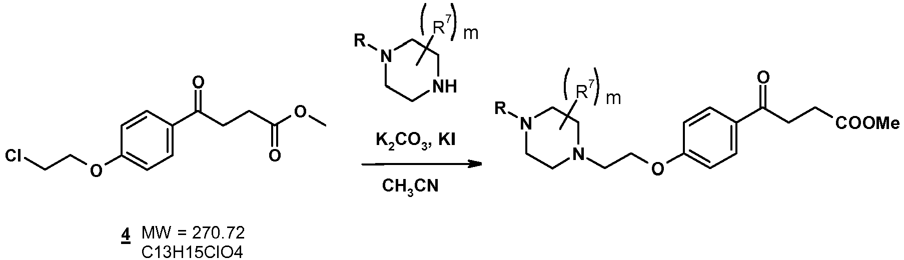

Equipement : Appareil Stem équipé d'un système de chauffage et d'une agitation orbitalaire, Réacteurs de 9ml - Manifold - système d'évaporation multivac - Appareil pour extraction Allexis

Sous balayage d'azote, le dérivé chloré **4** (500mg, 1,85mmol, 1éq.) est réparti dans les différents réacteurs puis mis en solution dans l'acétonitrile (5ml) en présence de R-pipérazine (1,85mmol, 1éq.), de carbonate de potassium (766mg, 5,54mmol, 3éq.) préalablement séché, de iodure de potassium (307mg, 1,85mmol, 1éq.). Après avoir réalisé un balayage d'azote, les réacteurs sont fermés et chauffés à 80°C.

Après 72 heures, le chauffage est arrêté. Après retour à température ambiante, les différents milieux réactionnels sont filtrés en parallèle sur cartouche Supelco reliée à un Manifold pour éliminer les sels inorganiques. Après rinçage par de l'acétonitrile, les filtrats sont concentrés à sec sous pression réduite à l'aide du multivac. Les résidus obtenus sont repris par de l'eau (20ml) et extraits trois fois, en parallèle sur appareil Allexis, par de l'acétate d'éthyle (10ml), Les différentes phases organiques rassemblées sont séchées sur sulfate de magnésium, filtrées puis concentrées sous pression réduite à l'aide du multivac.

Les différents bruts réactionnels sont purifiés par chromatographie sur colonne prépackée Redisep 40g, système Biotage SP4, en utilisant un gradient dichlorométhane / méthanol.

### Etape 5 : Hydrolyse acide

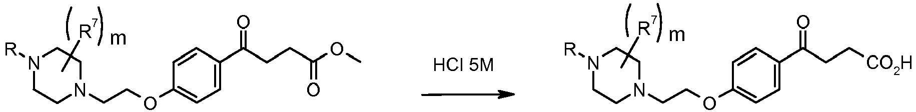

Equipement : Syncore - Système de chauffage - agitation orbitalaire - Réacteurs de 50ml

Les différents esters sont mis en solution dans l'acide chlorhydrique 5 puis les solutions sont chauffées à 90°C pendant 2 heures. Un contrôle par CCM (Eluant : dichlorométhane/Méthanol 98/2, révélation UV) du milieu réactionnel de chaque réacteur permet de vérifier la disparition des esters au profit de produits plus polaires.

Les milieux réactionnels sont concentrés à sec sous pression réduite au multivac. Les solides obtenus sont triturés avec de l'acétone (10V) puis filtrés sur fritté et séchés sous vide.

### Etape 5 : Saponification

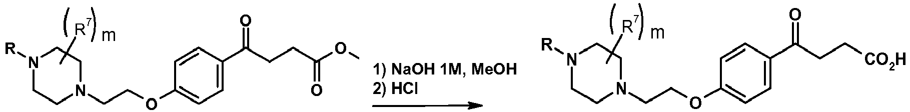

Equipement : Radley équipé de réacteurs et muni d'une agitation magnétique.

Les différents esters sont mis en solution dans le méthanol (4V) en présence d'une solution de soude 1M (8V). Après une nuit d'agitation dans ces conditions, un contrôle par CCM (Eluant : dichlorométhane/Méthanol 98/2, révélation UV) permet de vérifier la disparition des esters au profit de produits plus polaires.

Après concentration du méthanol, les différentes solutions aqueuses sont extraites par de l'acétate d'éthyle (5ml) puis acidifiées à pH 1 à l'aide d'une solution d'acide chlorhydrique 3M.

Les acides sont ensuite isolés par désalification en réalisant une élution des différentes solutions aqueuses sur résine Porapak Rxn.

Protocole de désalification :
- Conditionnement de la résine par 45ml de méthanol
- Déposer la phase aqueuse (4 à 5ml)
- Rincer par 100ml de méthanol
- Eluer par 100ml d'une solution d'ammoniaque 2M dans le méthanol
- Concentrer à sec sous pression réduite

Les solides obtenus après désalification sont triturés par de l'éther éthylique ou recristallisés dans l'acétonitrile.

Les composés suivants ont été obtenus par mise en oeuvre du schéma général de l'exemple 1 (étape 5 hydrolyse acide)

| Rendement : 95% Structure : | Composé 1 | |
|---|---|---|
| 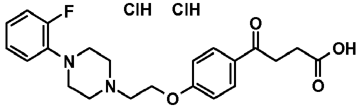 | | |
| | Masse moléculaire : | 473,38 |
| | Formule brute : | C₂₂H₂₇Cl₂FN₂O₄ |
| | Forme/couleur : | Solide blanc |
| RMN-1H (DMSO, 300MHz, δ ppm) : 2.56 (t, 2H); 3.18-3.41 (m, 6H); 3.50 (d, 2H); 3.63-3.70 (m, 4H); 4.59 (t, 2H); 7.00-7.23 (m, 6H); 7.99 (d, 2H); 11.49 (s large, 1H) | | |
| MS (ESI) : 401,10 (MH⁺); 399,2 (MH⁻) forme base | | |

| Rendement : 93% Structure : | Composé 2 | |
|---|---|---|
| 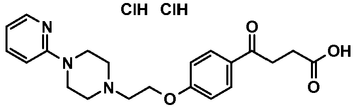 | | |
| | Masse moléculaire : | 456,37 |
| | Formule brute : | C₂₁ H₂₇Cl₂N₃O₄ |
| | Forme/couleur : | Solide blanc |
| RMN-1H (DMSO, 300MHz, δ ppm) : 2.56 (t, 2H); 3.18-3.32 (m, 4H); 3.42-3.52 (m, 2H); 3.62-3.72 (m, 4H); 4.45 (d, 2H); 4.55 (t, 2H); 6.88 (t, 1H); 7.12-7.18 (m, 3H); 7.80-7.85 (m, 1H); 8.00 (d, 2H); 8.14 (d, 1H); 11.24 (s large, 1H) | | |
| MS (ESI) : 384,30 (MH⁺); 382,20 (MH⁻) forme base | | |

| Rendement : 99% Structure : | Composé 3 | |
|---|---|---|
| 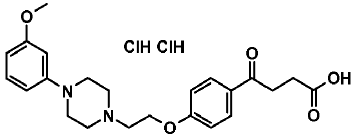 | | |
| | Masse moléculaire : | 485,41 |
| | Formule brute : | C₂₃H₃₀Cl₂N₂O₅ |
| | Forme/couleur : | Solide blanc |
| RMN-1H (DMSO, 300MHz, δ ppm) : 2.56 (t, 2H); 3.12-3.33 (m, 6H); 3.61-3.67 (m, 4H); 3.73 (s, 3H); 3.84 (d, 2H); 4.57 (t, 2H); 6.45 (dd, 1H); 6.53 (s, 1H); 6.58 (dd, 1H); 7.10-7.19 (m, 3H); 8.00 (d, 2H); 11.21 (s large, 1H) | | |
| MS (ESI) : 413,20 (MH⁺); 411,20 (MH⁻) forme base | | |

### Exemple 2. Schéma générale de préparation des composés pour lesquels R¹ représente -(CH₂)₄C(O)OH

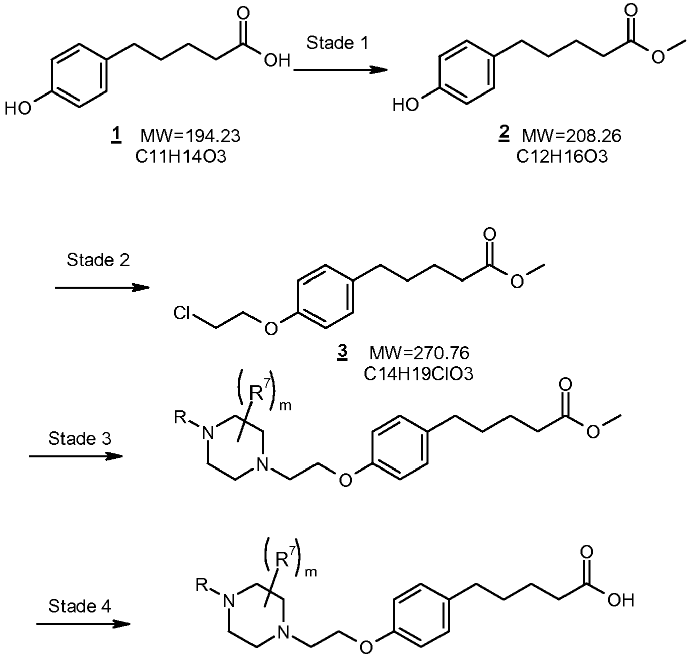

L'Etape 1 est une réaction d'estérification, les étapes 2 et 3 sont des réactions de substitutions nucléophiles et l'étape 4 est une réaction d'hydrolyse.

### Etape 1 : Estérification

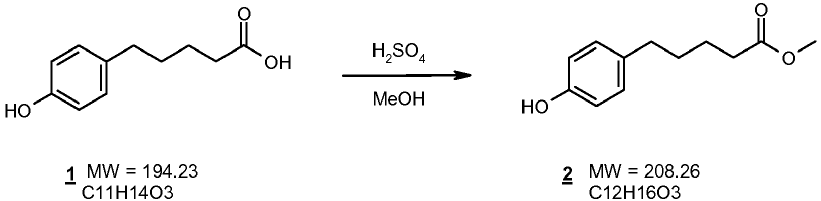

Equipement : Tricol de 500ml équipé d'une agitation magnétique, d'un réfrigérant et placé sous balayage d'azote - Bain d'huile.

L'acide 5-(4-hydroxyphényl)-pentanoique 1 (25g) est mis en solution dans le méthanol (375ml) avant de couler lentement une solution d'acide sulfurique (25ml). La solution ainsi obtenue est chauffée à 65°C pour la nuit.

L'avancement de la réaction est contrôlé par CCM (éluant : Heptane/Acétate d'éthyle : 1/1). Après une nuit d'agitation dans ces conditions, on observe la disparition de l'acide de départ 1 au profit d'un produit moins polaire.

Le milieu réactionnel est concentré à sec sous pression réduite. Le résidu obtenu est repris par du dichlorométhane (300ml). Le milieu hétérogène est neutralisé et basifié, avec précaution, avec une solution saturée d'hydrogénocarbonate de sodium (pH 8-9). La phase aqueuse est ensuite extraite trois fois par du dichlorométhane. Une fois rassemblées, les phases organiques sont lavées une fois par une solution de chlorure de sodium saturée, séchées sur sulfate de magnésium, filtrées puis concentrées sous pression réduite pour fournir une huile rosée (27g).
Rendement 99%

### Etape 2 : Substitution nucléophile

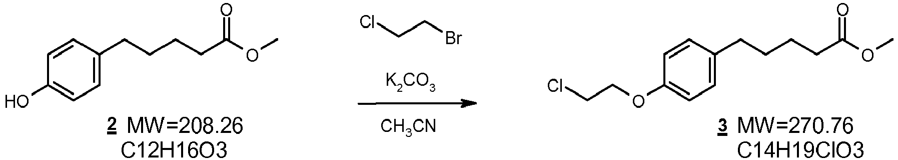

Equipement : Tricol de 500ml équipé d'une agitation magnétique, d'un réfrigérant et placé sous balayage d'azote - Bain d'huile.

L'ester 5-(4-hydroxyphényl)-pentanoate de méthyle 2 (26.8g) est mis en solution dans l'acétonitrile (250ml). Le carbonate de potassium (53.36g), préalablement séché, est ajouté à la solution. Le milieu réactionnel est chauffé à 50°C avant de couler lentement une solution de 1-bromo-2-chloroéthane (55.36g) dans l'acétonitrile (60ml). Le milieu réactionnel est chauffé à 80°C pour la nuit.

L'avancement de la réaction est contrôlé par CCM (éluant : Heptane/Acétate d'éthyle : 7/3). Après 24H d'agitation dans ces conditions, on observe toujours la présence du phénol 2. On ajoute une quantité supplémentaire de 1-bromo-2-chloroéthane (9.23g, 64.3mmol, 0.5éq.) et on maintient le milieu réactionnel sous agitation à 80°C pendant encore 24H. Un contrôle CCM montre que la réaction n'évolue pas. Une RMN du proton d'un aliquot du milieu réactionnel permet de quantifier les composés 2 et 3 selon un ratio de l'ordre de 33/66.

Après retour à température ambiante, le milieu réactionnel est filtré pour éliminer le carbonate de potassium. Le carbonate de potassium est rincé par de l'acétate d'éthyle puis le filtrat est concentré à sec sous pression réduite. Le résidu obtenu est repris par de l'eau (100ml) et la phase aqueuse est extraite trois fois par de l'acétate d'éthyle (100ml). Les phases organiques sont rassemblées, lavées avec une solution d'hydroxyde de sodium 1M (200ml) puis à l'eau (100ml), séchées sur sulfate de magnésium, filtrées puis concentrées sous pression réduite pour fournir une huile beige (39.5g).

L'analyse RMN-1H du brut réactionnel révèle la présence du dérivé chloré 3 attendu en mélange avec près de 30% de matière première 2.

Le brut réactionnel est purifié par chromatographie sur gel de silice (1L). Les composés à séparer sont élués en utilisant un gradient heptane / acétate d'éthyle.

Le composé souhaité est isolé sous la forme d'une huile (22.4g).
Rendement 64%

### Etape 3 : Substitution nucléophile

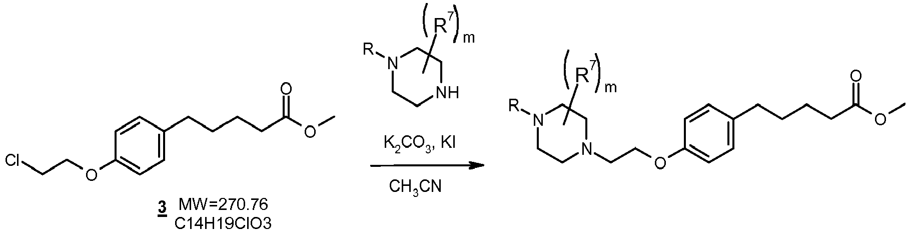

Equipement : Appareil Stem équipé d'un système de chauffage et d'une agitation orbitalaire, Réacteurs de 9ml - Manifold - système d'évaporation multivac - Appareil pour extraction Allexis

Sous balayage d'azote, le dérivé chloré 3 (500mg, 1.85mmol, 1éq.) est réparti dans les différents réacteurs puis mis en solution dans l'acétonitrile (5ml) en présence de R-pipérazine (1.85mmol, 1éq.), de carbonate de potassium (766mg, 5.54mmol, 3éq.) préalablement séché et de iodure de potassium (307mg, 1.85mmol, 1éq.). Après avoir réalisé un balayage d'azote, les réacteurs sont fermés et chauffés à 80°C.

Après 72 heures, le chauffage est arrêté. Après retour à température ambiante, les différents milieux réactionnels sont filtrés en parallèle sur cartouche Supelco reliée à un Manifold pour éliminer les sels inorganiques. Après rinçage par de l'acétonitrile, les filtrats sont concentrés à sec sous pression réduite à l'aide du multivac. Les résidus obtenus sont repris par de l'eau (20ml) et extraits trois fois, en parallèle sur appareil Allexis, par de l'acétate d'éthyle (10ml). Les différentes phases organiques rassemblées sont séchées sur sulfate de magnésium, filtrées puis concentrées sous pression réduite à l'aide du multivac.

Les différents bruts réactionnels sont purifiés par chromatographie sur colonne prépackée Redisep 40g, système Biotage SP4, en utilisant un gradient dichlorométhane / méthanol.

### Etape 4 : Hydrolyse

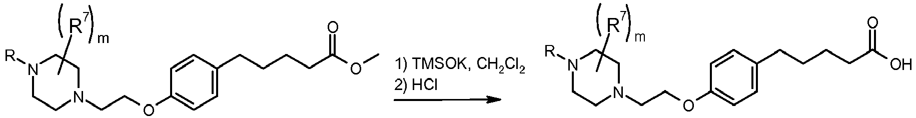

Equipement : Appareil Stem équipé d'un système de chauffage et d'une agitation orbitalaire, Réacteurs de 9ml - Système d'évaporation multivac

Les différents esters issus de 3 sont mis en solution dans le dichlorométhane (5ml) en présence de triméthylsilanoate de potassium. Le milieu réactionnel est chauffé à 35°C pour la nuit.

Après une nuit d'agitation dans ces conditions, un contrôle par CCM (Eluant : dichlorométhane/Méthanol 98/2, révélation UV) permet de vérifier la disparition des esters au profit de produits plus polaires.

Les différents milieux réactionnels sont concentrés à sec sous pression réduite. Les résidus obtenus sont triturés par un mélange de diéthyle éther (4 volumes) et d'éthanol (2 volumes) pour éliminer l'excès de triméthylsilanoate de potassium et le triméthylméthoxysilane résiduel. Après filtration les carboxylates de potassium sont isolés et séchés sous pression réduite.

Chaque carboxylate de potassium est mis en solution dans un minimum d'eau distillée (1 à 11ml) avant d'additionner une solution d'acide chlorhydrique 1N (2éq.). Après 30 minutes d'agitation, les acides formés précipitent sous la forme d'une gomme. Chaque acide est trituré régulièrement jusqu'à l'apparition d'un précipité poudreux. Les précipités sont filtrés, lavés une fois par de l'eau puis séchés sous pression réduite en présence de P₂O₅.

Les composés suivants ont été obtenus par mise en oeuvre du schéma général de l'exemple 2

| Rendement : 85% Structure : | | |
|---|---|---|
| 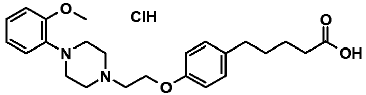 | Composé 4 | |
| | Masse moléculaire : | 448.99 |
| | Formule brute : | C₂₄H₃₃ClN₂O₄ |
| | Forme/couleur : | Solide blanc |
| ppm)RMN-1H (MeOD, 300MHz, δ : 1.47-1.59 (m, 4H); 2.23 (t, 2H); 2.53 (t, 2H); 3.19-3.62 (m, 10H); 3.80 (s, 3H); 4.33 (t, 2H); 6.84-7.04 (m, 6H); 7.09 (d, 2H) | | |
| MS (ESI) : 413.20 (MH⁺); 411.20 (MH⁻) | | |

### Exemple 3. Etude de l'activité antidiabétique additive entre la metformine et divers composés du porte-feuille de Metabolys chez la souris Swiss

On a déterminé l'activité antidiabétique des composés des exemples 1 et 2 par voie orale chez la souris Swiss normale à jeun pendant 17 heures.

Les souris sont utilisées à l'âge de 8 semaines. La stabulation des animaux est réalisée au minimum pendant une semaine après réception (commande chez Charles River France) et jusqu'au jour de l'expérimentation et dans une animalerie à température régulée à 21-22 °C et soumis à un cycle de lumière (de 9h à 17 h) et d'obscurité (de 19h à 7h). Leur alimentation a consisté en un régime d'entretien; eau et nourriture ont été fournies « ad libitum ».

Les animaux sont traités par voie orale 1 heure avant l'administration orale d'une charge en glucose (test de tolérance oral au glucose ou OGTT en anglais). La glycémie à été mesurée à divers temps avant et après cette charge en glucose. Cette glycémie est mesurée à l'aide d'un glucomètre (Lifescan OneTouch Ultra, Lifescan, Johnson and Johnson Company) et de bandelettes de mesure de glycémie OneTouch Ultra.

Dans les tableaux ci-dessous, n représente le nombre de souris incluses dans l'essai.

### 1. Etude de l'effet additif du composé 1 (50 mg/kg per os) avec l'effet de la metformine (300 mg/kg per os) sur l'évolution de la glycémie suite à une charge orale en glucose (OGTT) chez des souris Swiss à jeun depuis 17 heures.

**Tableau 1. Effet additif avec la metformine du composé 1 sur la glycémie (mM/litre) de souris Swiss à jeun 17h au cours d'un test de tolérance au glucose.**

| Condition expérimentale | Glycémie | | | | | |
|---|---|---|---|---|---|---|
| Temps (mn) | -60 | 0 | 30 | 60 | 90 | 120 |
| Véhicule (n = 5) | 4,4 ±0,3 | 4,8 ± 0,4 | 19,5 ± 1,2 | 15,1 ± 1,5 | 10,1 ± 0,9 | 7,8 ± 0,8 |
| Composé 1 (50 mg/kg) (n = 5) | 5,0 ± 0.2 | 4,5 ± 0,3 | 15,2 ± 1,9 | 10,6 ± 0,2 | 6,5 ± 0,5 | 5,3 ± 0,2 |
| Metformine (300 mg/kg) (n = 5) | 3,7 ± 0,3 | 3,2 ± 0,3 | 8,6 ± 0,5 | 9,4 ± 0,8 | 7,7 ± 1.0 | 6,2 ± 0,8 |
| Composé 1 (50 mg/kg)+ Metformine (300 mg/kg) (n = 5) | 3,9 ± 0,2 | 3,0 ± 0,3 | 7,8 ± 1,0 | 4,5 ± 0,8 | 3,8 ± 0,6 | 2,6 ± 0,6 |

Les 2 composés ont été administrés successivement une heure avant la charge en glucose. Tous les groupes étaient composés de 5 souris (metformine seule, composé 1 seul, metformine + composé 1, groupe témoin avec le véhicule seul). Le sang a été prélevé à 0, 30, 60, 90 et 120 minutes après la charge en glucose.

Après la charge en glucose, la metformine seule a fait chuter l'aire sous la courbe de la glycémie de 44,2 %, le composé 1 seul de 42,5 % et la combinaison metformine plus composé 1 de 79,5 %.

Ces résultats montrent un effet important des compositions de l'invention sur l'évolution de la glycémie.

### 2. Etude de l'effet additif du composé 2 (50 mg/kg per os) avec l'effet de la metformine (300 mg/kg per os) sur l'évolution de la glycémie suite à une charge orale en glucose (OGTT) chez des souris Swiss à jeun depuis 17 heures.

**Tableau 2. Effet additif avec la metformine du composé 2 sur la glycémie (mM/litre) de souris Swiss à jeun 17h au cours d'un test de tolérance au glucose**

| Condition expérimentale | Glycémie | | | | | |
|---|---|---|---|---|---|---|
| Temps (mn) | -60 | 0 | 30 | 60 | 90 | 120 |
| Véhicule (n = 4) | 4,4 ±0,2 | 5,2 ± 0,4 | 19,6 ± 2,9 | 18,7 ± 2,9 | 9,3 ± 1,2 | 7,1 ± 0,6 |
| Composé 2 (50 mg/kg) (n = 4) | 4,6 ± 0,4 | 4,9 ± 0,2 | 14,5 ± 1,4 | 10,4 ± 0,5 | 8,3 ± 1,1 | 6,8 ± 0,6 |
| Metformine (300 mg/kg) (n = 4) | 3,8 ± 0,1 | 3,6 ± 0,3 | 11,2 ± 0,3 | 9,5 ± 0,4 | 8,0 ± 1,3 | 8,2 ± 0,7 |
| Composé 2 (50mg/kg)+ Metformine (300 mg/kg) (n = 4) | 4,5 ± 0,5 | 3,8 ± 0,3 | 7,3 ± 1,8 | 5,9 ± 1,8 | 4,9 ± 1,3 | 4,1 ± 0,7 |

Les 2 composés ont été administrés successivement une heure avant la charge en glucose. Tous les groupes étaient composés de 4 souris (metformine seule, composé 2 seul, metformine + composé 2, groupe témoin avec le véhicule seul). Le sang a été prélevé à 0, 30, 60, 90 et 120 minutes après la charge en glucose.

Après la charge en glucose, la metformine seule a fait chuter l'aire sous la courbe de la glycémie de 38,7 %, le composé 2 seul de 40,5 % et la combinaison metformine plus composé 2 de 76,8 %.

Ces résultats montrent un effet important des compositions de l'invention sur l'évolution de la glycémie.

### 3. Etude de l'effet additif du composé 3 (50 mg/kg per os) avec l'effet de la metformine (300 mg/kg per os) sur l'évolution de la glycémie suite à une charge orale en glucose (OGTT) chez des souris Swiss à jeun depuis 17 heures.

**Tableau 3 Effet additif avec la metformine du composé 3 sur la glycémie (mM/litre) de souris Swiss à jeun 17h au cours d'un test de tolérance au glucose**

| Condition expérimentale | Glycémie | | | | | |
|---|---|---|---|---|---|---|
| Temps (mn) | -60 | 0 | 30 | 60 | 90 | 120 |
| Véhicule (n = 4) | 4,7 ±0,4 | 5,4 ± 0,3 | 17,2 ± 2,1 | 15,4 ± 2,1 | 10,5 ± 1,4 | 8,3 ± 0,7 |
| Composé 3 (50 mg/kg) (n = 4) | 4,4 ± 0,3 | 5,5 ± 0,3 | 11,3 ± 0,6 | 9,7 ± 0,4 | 7,4 ± 0,3 | 7,2 ± 0,5 |
| Metformine (300 mg/kg) (n = 4) | 5,6 ± 0,3 | 6,0 ± 0,3 | 9,2 ± 0,8 | 8,2 ± 0,4 | 7,7 ± 0,2 | 9,6 ± 0,8 |
| Composé 3 (50 mg/kg)+ Metformine (300 mg/kg) (n = 4) | 5,4 ± 0,4 | 5,4 ± 0,3 | 8,4 ± 0,7 | 7,7 ± 0,7 | 7,0 ± 1,5 | 6,5 ± 1,2 |

Les 2 composés ont été administrés successivement une heure avant la charge en glucose. Tous les groupes étaient composés de 5 souris (metformine seule, composé 3 seul, metformine + composé 3, groupe témoin avec le véhicule seul). Le sang a été prélevé à 0, 30, 60, 90 et 120 minutes après la charge en glucose.

Après la charge en glucose, la metformine seule a fait chuter l'aire sous la courbe de la glycémie de 68,5 %, le composé 3 seul de 54,9 % et la combinaison metformine plus composé 3 de 74,4 %.

Ces résultats montrent un effet important des compositions de l'invention sur l'évolution de la glycémie.

### 4. Etude de l'effet additif du composé 4 (50 mg/kg per os) avec l'effet de la metformine (300 mg/kg per os) sur l'évolution de la glycémie suite à une charge orale en glucose (OGTT) chez des souris Swiss à jeun depuis 17 heures.

**Tableau 4. Effet additif avec la metformine du composé 4 sur la glycémie (mM/litre) de souris Swiss à jeun 17h au cours d'un test de tolérance au glucose.**

| Condition expérimentale | Glycémie | | | | | |
|---|---|---|---|---|---|---|
| Temps (mn) | -60 | 0 | 30 | 60 | 90 | 120 |
| Véhicule (n = 4) | 3,7 ±0,2 | 4,5 ± 0,2 | 20,2 ± 2,6 | 18,0 ± 1,3 | 10,4 ± 0,2 | 7,8 ± 0,5 |
| Composé 4 (50 mg/kg) (n = 4) | 3,4 ± 0,2 | 4,0 ± 0,3 | 18,6 ± 3,2 | 13,3 ± 1,5 | 8,8 ± 1,0 | 6,8 ± 1,0 |
| Metformine (300 mg/kg) (n = 4) | 4,2 ± 0,3 | 3,6 ± 0,3 | 9,5 ± 0,6 | 11,2 ± 1,8 | 9,6 ± 0,9 | 8,6 ± 0,7 |
| Composé 4 (50 mg/kg)+ Metformine (300 mg/kg) (n = 4) | 3,5 ± 0,3 | 3,3 ± 0,1 | 8,3 ± 1,2 | 5,5 ± 1,4 | 4,8 ± 1,1 | 4,5 ± 0,9 |

Les 2 composés ont été administrés successivement une heure avant la charge en glucose. Tous les groupes étaient composés de 4 souris (metformine seule, composé 4 seul, metformine + composé 4, groupe témoin avec le véhicule seul). Le sang a été prélevé à 0, 30, 60, 90 et 120 minutes après la charge en glucose.

Après la charge en glucose, la metformine seule a fait chuter l'aire sous la courbe de la glycémie de 54,3 %, le composé 4 seul de 18,7 % et la combinaison metformine plus composé 4 de 76,4%. L'effet est totalement additif.

Ces résultats montrent un effet important des compositions de l'invention sur l'évolution de la glycémie.

### Exemple 4 : Etude de l'impact du composé 2 sur le foie, le muscle squelettique et le pancréas

### A) Au niveau du foie

Des cellules hépatiques de rats à jeun depuis 48 heures ont été incubées avec du lactate ou de la glutamine en absence et en présence de 2mM du composé 2. Un dosage enzymatique de la consommation de lactate et de glutamine a été réalisé, la production de leurs métabolites a également été analysée. Enfin, la concentration cellulaire en ATP a été déterminée.

Les résultats sont regroupés dans le tableau 5 ci-dessous.

**Tableau 5. Effets du composé 2 (2 mM) sur le métabolisme du lactate et de la glutamine dans des cellules hépatiques de rats à jeun depuis 48h**

| Condition expérimentale | Consommation de substrat (-) | Production de glucose | Oxydation complète | Concentration d'ATP | Transport mitochondrial de NADH |
|---|---|---|---|---|---|
| 5 mM lactate | -4,49 ± 0,31 | 2,00 ± 0,13 | 0,04 ± 0,26 | 59,2 ± 12,0 | 0,48 ± 0,25 |
| 5 mM lactate + 2 mM composé 2 | -2,59 ± 0,34* | 0,76 ± 0,13* | 0,64 ± 0,30* | 31,8 ± 8,0* | 1,06 ± 0,33* |
| 5 mM glutamine | -1,79 ± 0,23 | 1,13 ± 0,17 | -0,78 ± 0,42 | 70,8 ± 11,3 | - |
| 5 mM glutamine + 2 mM composé 2 | -2,77 ± 0,15* | 0,78 ± 0,09* | 0,59 ± 0,35* | 34,2 ± 7,6* | - |

| | | | | | |
|---|---|---|---|---|---|
| *p < 0,05, test de Student pour données appariées ; moyenne ± S.E.M. ; n = 6 ; µmoles / fiole sauf ATP (nanomoles pour 2 tranches de foie coupées avec précision) ; oxydation complète : pour lactate = consommation de lactate - (2 ^{∗} glucose + pyruvate + alanine produits), pour glutamine = consommation de glutamine - (2 ^{∗} glucose + pyruvate + lactate + alanine + glutamate produits) ; transport mitochondrial de NADH tel qu'indiqué ci-dessus. | | | | | |

Les résultats montrent que le composé 2 stimule l'oxydation complète du lactate et de la glutamine sans augmenter le niveau cellulaire d'ATP mais, au contraire, en le diminuant. Cela suggère qu'il y a découplage entre l'oxydation des substrats (lactate et glutamine) qui est augmentée et la phosphorylation de l'ADP en ATP qui est diminuée alors que ces 2 processus sont normalement couplés, c'est-à-dire qu'ils évoluent normalement dans le même sens. Le fait que l'oxydation des substrats est augmentée, c'est-à-dire que le flux d'électrons au niveau de la chaîne respiratoire est accru, est également attesté par une augmentation du transport intramitochondrial de NADH ; ce transport est égal à la différence entre la consommation de lactate (la consommation d'une molécule de lactate produit une molécule de NADH) et 2 fois la production de glucose (car la synthèse d'une molécule de glucose nécessite 2 molécules de NADH).

### Etude du métabolisme du lactate

Compte tenu du fait qu'une concentration de 2 mM de composé 2 conduit à une forte inhibition de la consommation de lactate (tableau 5), une concentration plus faible de composé 2 (0,5 mM) a été utilisée pour démontrer une augmentation de l'oxydation complète de ces substrats (lactate et glutamine) par ce composé. Pour faire une telle démonstration, la même concentration (5 mM) du 1-, 2- et 3-13C-lactate a été utilisée et la consommation de lactate et la production de 13CO2 en utilisant la RMN quantitative du carbone 13 a été mesurée. L'oxydation complète d'un substrat est mesurée par la valeur la plus faible de CO2 produite à partir de l'un de ses carbones. Cela a pour conséquence que ce carbone se retrouve en plus grande quantité dans les produits non volatils (autres que le CO2) formés au cours du métabolisme de ce substrat.

Le tableau 6 ci-dessous montre que les produits non volatils formés au cours du métabolisme du lactate sont davantage marqués lorsqu'on utilise du 2-13C-lactate comme substrat. L'oxydation complète du lactate peut donc être déterminée en mesurant la production du 13CO2 à partir du 2-13C-lactate.

**Tableau 6. Somme des marquages des produits non volatils du métabolisme du lactate dans des cellules hépatiques de rats à jeun depuis 48h**

| Condition expérimentale | 1-13C-lactate | 2-13C-lactate | 3-13C-lactate |
|---|---|---|---|
| 5 mM lactate | 2,28 ± 0,05+ | 6,97 ± 0,10 | 5,92 ± 0,14+ |
| 5 mM lactate + 0,5 mM composé 2 | 1,36 ± 0,09*+ | 4,94 ± 0,15* | 4,06 ± 0,15*+ |

| | | | |
|---|---|---|---|
| *p < 0,05 (différent du témoin [lactate seul]), + p < 0,05 (différent du 2-13C-lactate), test t de Student pour données appariées ; moyenne ± S.E.M. ; n = 3 ; µmoles de carbone 13 / fiole. | | | |

Le tableau 7 ci-dessous montre que le composé 2 stimule l'oxydation complète du lactate bien qu'il diminue légèrement (de 10%) la consommation de ce substrat; cet effet s'accompagne d'une diminution du niveau cellulaire d'ATP, une molécule indispensable à la synthèse de glucose à partir de lactate. Il est donc démontré un découplage de la phosphorylation oxydative.

**Tableau 7. Effet du MTBL0036 (0,5 mM) sur la production de 13CO2 et de glucose à partir du 2-13C-lactate (5 mM) et la concentration cellulaire d'ATP dans des cellules hépatiques de rats à jeun depuis 48h**

| Condition expérimentale | Production de glucose | Production de 13CO2 | Concentration d'ATP |
|---|---|---|---|
| 5 mM 2-13C-lactate | 6,17 ± 0,15 | 5,18 ± 0,19 | 243,6 ± 37,4 |
| 5 mM 2-13C-lactate + 0,5 mM composé 2 | 4,69 ±0,14* | 5,99 ± 0,09* | 188,4 ± 25,2* |

| | | | |
|---|---|---|---|
| *p < 0,05 (différent du témoin [lactate seul]), test t de Student pour données appariées ; moyenne ± S.E.M. ; n = 4 ; µmoles / fiole sauf pour l'ATP (nanomoles pour 6 tranches de foie coupées avec précision). | | | |

### Différences entre le mécanisme d'action du composé 2 et celui de la metformine :

Il doit être rappelé ici que le lactate est le principal substrat de la gluconéogenèse hépatique.

Il est bien établi que la metformine est un inhibiteur modéré du complexe I de la chaîne respiratoire, ce qui conduit à une activation de l'AMPK et aux effets métaboliques qui en résultent, notamment une inhibition de la gluconéogenèse hépatique. Dans des cellules hépatiques de rat, contrairement au composé 2, la metformine diminue la production de 13CO2 à partir de 2-13C-lactate ainsi que la synthèse de glucose.

A l'inverse, le composé 2 est un activateur de la chaîne respiratoire suite à un effet découplant modéré de la phosphorylation oxydative. Ce composé a donc le potentiel de corriger le déficit mitochondrial existant dans le diabète de type 2 en augmentant l'oxydation des substrats et, simultanément, en diminuant la synthèse hépatique de glucose.

### Etude du métabolisme du lactate

L'oxydation complète de la glutamine peut être étudiée en mesurant la production de 13CO2 à partir de 3-13C-glutamine (Biochem. J., 2004, 378, 485-495).

Le tableau 8 ci-dessous montre que le composé 2 augmente la production de 13CO2 à partir de la 3-13C-glutamine sans augmenter la concentration cellulaire d'ATP.

**Tableau 8. Effet du composé 2 (0,5 mM) sur la production de glucose, l'oxydation complète de la glutamine et le niveau cellulaire d'ATP dans des cellules hépatiques de rats à jeun depuis 48h**

| Condition expérimentale | Production de glucose | Production de 13CO2 | Concentration d'ATP |
|---|---|---|---|
| 5 mM 3-13C-glutamine | 6,01 ± 0,09 | 0,23 ± 0,40 | 356,1 ± 41,4 |
| 5 mM 3-13C-glutamine + 0,5 mM composé 2 | 6,05 ± 0,30 (NS) | 1,86 ± 0,28* | 362,9 ± 53,3 (NS) |

| | | | |
|---|---|---|---|
| *p < 0,05 (différent du témoin [glutamine seule]), test ANOVA suivi du test Newman Keuls ; moyenne ± S.E.M.; n = 4 expériences; µmoles / fiole sauf pour l'ATP (nanomoles pour 6 tranches de foie coupées avec précision). NS = statistiquement non significatif. | | | |

### B) Au niveau du muscle squelettique et du pancréas

La sécrétion d'insuline chez 6 rats STZ-N0a été observée par rapport à l'administration du composé 2 (200 mg/kg) avant (temps -60 min et -30 min) et après une charge en glucose. (temps 0 min) Il est noté une augmentation de la sécrétion d'insuline uniquement quand le composé 2 provoque une augmentation de la consommation périphérique de glucose par le muscle squelettique (Tableaux 9 à 12 qui représentent une moyenne des résultats obtenus). En effet, Les tableaux 9 et 10 montrent que l'augmentation de la consommation de glucose dans le muscle (réduction de l'AUC [Area Under the Curve]) s'accompagne d'une augmentation du niveau circulant d'insuline. Les tableaux 11 et 12 montrent qu'en l'absence d'augmentation de consommation de glucose dans le muscle il n'y a pas d'augmentation du niveau circulant d'insuline.

Les 2 évènements étant liés on peut donc en conclure que l'augmentation de la consommation de glucose par le muscle squelettique est due à la stimulation de la sécrétion d'insuline dont le mécanisme d'action est bien connu.

**Tableau 9 Effets du composé 2 (200 mg/kg) sur la glycémie**

| Condition expérimentale | Glycémie (mmol/l) | | | | | | |
|---|---|---|---|---|---|---|---|
| Temps (mn) | -60 | 0 | 30 | 60 | 90 | 120 | AUC |
| témoin (n = 6) | 4,62 | 4,83 | 11,63 | 17,38 | 16,33 | 12,98 | 1048 |
| Composé 2 (200 mg/kg) (n = 6) | 4,75 | 8,75 | 12,97 | 13,15 | 11,37 | 10,13 | 356 |

**Tableau 10 Effets du composé 2 (200 mg/kg) sur la concentration circulante d'insuline chez des rats**

| Condition expérimentale | Insuline (ng/ml) | | | | | |
|---|---|---|---|---|---|---|
| Temps (mn) | -60 | 0 | 30 | 60 | 90 | 120 |
| témoin (n = 6) | 0,47 | 0,41 | 0,70 | 0,59 | 0,54 | 0,53 |
| Composé 2 (200 mg/kg) (n = 6) | 0,47 | 0,41 | 0,70 | 0,59 | 0,54 | 0,53 |

**Tableau 11. Effets du composé 2 (25 mg/kg) sur la glycémie**

| Condition expérimentale | Glycémie (mmol/l) | | | | | | |
|---|---|---|---|---|---|---|---|
| Temps (mn) | -60 | 0 | 30 | 60 | 90 | 120 | AUC |
| témoin (n = 6) | 5,12 | 5,14 | 15,52 | 15,03 | 15,27 | 13,53 | 1025 |
| Composé 2 (200 mg/kg) (n = 6) | 5,12 | 5,23 | 11,15 | 16,21 | 14,36 | 15,33 | 1048 |

**Tableau 12. Effets du composé 2 (25 mg/kg) sur la concentration circulante d'insuline chez des rats**

| Condition expérimentale | Insuline (ng/ml) | | | | | |
|---|---|---|---|---|---|---|
| Temps (mn) | -60 | 0 | 30 | 60 | 90 | 120 |
| témoin (n = 6) | 1,5 | 1,16 | 2,17 | 1,79 | 1,34 | 1,33 |
| Composé 2 (200 mg/kg) (n = 6) | 1,11 | 1,41 | 1,76 | 1,48 | 1,61 | 1,61 |

### Différences entre le mécanisme d'action du composé 2 et celui de la metformine :

Il est bien établi qu'au niveau du muscle squelettique la metformine agit via l'activation de l'AMPK.

Le composé 2 agit différemment au niveau du muscle squelettique. Il agit via l'insuline dont la sécrétion est stimulée.

## Revendications

1. Composition comprenant de la metformine ou un sel de metformine, un véhicule ou excipient pharmaceutiquement acceptable et au moins un composé de formule (I) ses énantiomères, diastéréoisomères ou ses sels pharmaceutiquement acceptables 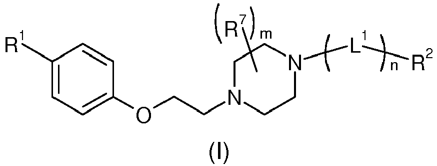 dans laquelle
▪ R¹ représente :
- un groupe -C(O)CR³R⁴CR⁵R⁶C(O)OH ;
- un groupe -(CH₂)₄C(O)OH ;
▪ m représente 0 ;
▪ n représente 0;
▪ R² représente :
- un groupe carbocycle en 5, 6 ou 7 membres, saturé, partiellement insaturé ou aromatique, non substitué ou substitué par un ou plusieurs substituants, identiques ou différents, notamment choisi parmi :
• un groupe alkoxy en C₁ à C₆, de préférence en C₁ à C₃, linéaire ou ramifié, par exemple méthoxy, éthoxy, propoxy, iso-propoxy, butoxy, ter-butoxy ;
• un atome d'halogène, par exemple fluor, chlore, brome ;
• un groupe alkyle en C₁ à C₅, de préférence en C₁ à C₄, linéaire ou ramifié, par exemple méthyle, éthyle, propyle, butyle, iso-propyle, ter-butyle ;
• un groupe alkyle en C₁ à C₅, de préférence en C₁ à C₄, linéaire ou ramifié, substitué notamment par un ou plusieurs atomes d'halogène, par exemple trifluorométhyl ;
- un groupe hétérocycle en 5, 6 ou 7 membres, saturé, partiellement insaturé ou aromatique pouvant comprendre 1, 2 ou 3 hétéroatomes, identiques ou différents, notamment choisis parmi l'azote, l'oxygène ou le soufre, non substitué ou substitué par un ou plusieurs substituants, identiques ou différents, notamment choisi parmi :
• un groupe alkoxy en C₁ à C₆, de préférence en C₁ à C₃, linéaire ou ramifié, par exemple méthoxy, éthoxy, propoxy, iso-propoxy, butoxy, ter-butoxy ;
• un atome d'halogène, par exemple fluor, chlore, brome ;
• un groupe alkyle en C₁ à C₅, de préférence en C₁ à C₄, linéaire ou ramifié, par exemple méthyle, éthyle, propyle, butyle, iso-propyle, ter-butyle ; ou
• un groupe alkyle en C₁ à C₅, de préférence en C₁ à C₄, linéaire ou ramifié, substitué notamment par un ou plusieurs atomes d'halogène, par exemple trifluorométhyl.
▪ R³, R⁴, R⁵ et R⁶, identiques ou différents, représentent :
- un atome d'hydrogène ;
- un groupe alkyle, linéaire ou ramifié, en C₁ à C₅, de préférence en C₁ à C₄.

2. Composition selon la revendication 1, dans laquelle R² représente :
- un groupe carbocycle aromatique en 6 membres, de préférence phényle non substitué ou substitué par un ou plusieurs substituants, identiques ou différents, notamment choisi parmi :
• un groupe alkoxy en C₁ à C₆, de préférence en C₁ à C₃, linéaire ou ramifié, par exemple méthoxy, éthoxy, propoxy, iso-propoxy, butoxy, ter-butoxy ;
• un atome d'halogène, par exemple fluor, chlore, brome ;
• un groupe alkyle en C₁ à C₅, de préférence en C₁ à C₄, linéaire ou ramifié, par exemple méthyle, éthyle, propyle, butyle, iso-propyle, ter-butyle ;
• un groupe alkyle en C₁ à C₅, de préférence en C₁ à C₄, linéaire ou ramifié, substitué notamment par un ou plusieurs atomes d'halogène, par exemple trifluorométhyl ;
- un groupe hétérocycle aromatique en 5 ou 6 membres comprenant 1, 2 ou 3 hétéroatomes, identiques ou différents, notamment choisis parmi l'azote, le soufre et l'oxygène, de préférence pyridine, non substitué ou substitué par un ou plusieurs substituants, identiques ou différents, notamment choisi parmi :
• un groupe alkoxy en C₁ à C₆, de préférence en C₁ à C₃, linéaire ou ramifié, par exemple méthoxy, éthoxy, propoxy, iso-propoxy, butoxy, ter-butoxy ;
• un atome d'halogène, par exemple fluor, chlore, brome ;
• un groupe alkyle en C₁ à C₅, de préférence en C₁ à C₄, linéaire ou ramifié, par exemple méthyle, éthyle, propyle, butyle, iso-propyle, ter-butyle ;
• un groupe alkyle en C₁ à C₅, de préférence en C₁ à C₄, linéaire ou ramifié, substitué notamment par un ou plusieurs atomes d'halogène, par exemple trifluorométhyl.

3. Composition selon la revendication 1 ou 2, dans laquelle R³, R⁴, R⁵ et R⁶, identiques ou différents, représentent un atome d'hydrogène.

4. Composition selon l'une quelconque des revendications 1 à 3, dans laquelle R² représente :
- un groupe phényle non substitué ou substitué par un ou plusieurs substituants, identiques ou différents, notamment choisi parmi :
• un groupe alkoxy en C₁ à C₆, de préférence en C₁ à C₃, linéaire ou ramifié, par exemple méthoxy, éthoxy, propoxy, iso-propoxy, butoxy, ter-butoxy ;
• un atome d'halogène, par exemple fluor, chlore, brome ;
- un groupe hétérocycle aromatique en 5 ou 6 membres comprenant 1, 2 ou 3 hétéroatomes, identiques ou différents, notamment choisis parmi l'azote, le soufre et l'oxygène, de préférence pyridine.

5. Composition selon l'une quelconque des revendications 1 à 4, dans laquelle le composé de formule (I) est choisi parmi :

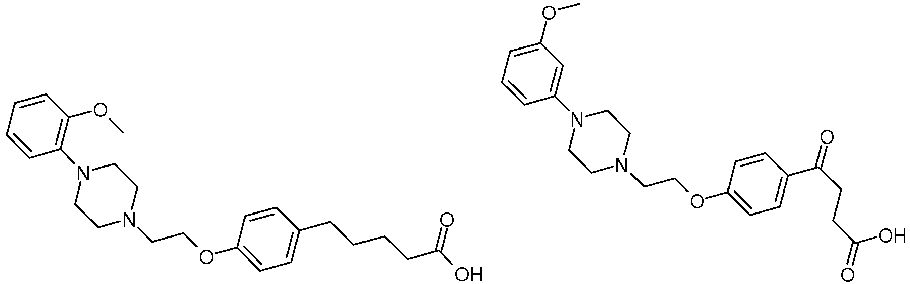

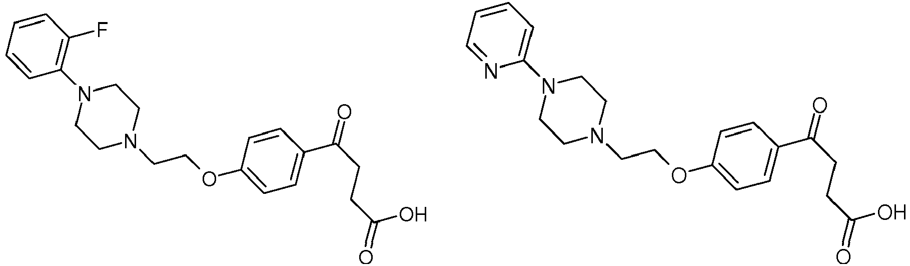

6. Composition selon l'une quelconque des revendications 1 à 5, pour son utilisation comme médicament.

7. Composition selon la revendication 6, pour son utilisation pour le traitement de pathologies associées au syndrome d'insulinorésistance.

8. Composition selon la revendication 7, pour son utilisation pour le traitement du diabète, notamment du diabète de type (II).

9. Composition selon la revendication 6, pour son utilisation pour l'inhibition de la néoglucogenèse.

10. Kit comprenant au moins un composé de formule (I) selon les revendications 1 à 5 ou un de ses sels pharmaceutiquement acceptable et de la metformine ou un sel de metformine pour une administration simultanée, séparée ou séquentielle à un patient qui en a besoin.

## Patentansprüche

1. Zusammensetzung, enthaltend Metformin oder ein Metforminsalz, eine pharmazeutisch annehmbarer Träger oder Hilfsstoff und mindestens eine Verbindung von Formel (I), seine Enantiomere, seine Diastereoisomere oder seine pharmazeutisch annehmbaren Salze 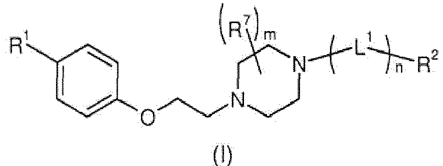 in der
▪ R¹ darstellt:
- eine Gruppe -C(O)CR³R⁴CR⁵R⁶C(O)OH;
- eine Gruppe -(CH₂)₄C(O)OH;
▪ m 0 darstellt;
▪ n 0 darstellt;
▪ R² darstellt:
- eine 5-, 6- oder 7-gliedrige carbocyclische Gruppe, gesättigt, teilweise ungesättigt oder aromatisch, unsubstituiert oder substituiert durch einen oder mehrere, gleiche oder verschiedene, Substituenten, insbesondere ausgewählt aus:
• eine C₁-C₆-Alkoxygruppe, vorzugsweise C₁-C₃, linear oder verzweigt, zum Beispiel Methoxy, Ethoxy, Propoxy, Iso-Propoxy, Butoxy, Ter-Butoxy;
• ein Halogenatom, zum Beispiel Fluor, Chlor, Brom;
• eine C₁-C₅-Alkylgruppe, vorzugsweise C₁-C₄, linear oder verzweigt, zum Beispiel Methyl, Ethyl, Propyl, Butyl, Isopropyl, Ter-Butyl;
• eine C₁-C₅-Alkylgruppe, vorzugsweise C₁-C₄, linear oder verzweigt, insbesondere substituiert durch ein oder mehrere Halogenatome, zum Beispiel Trifluormethyl;
- eine heterocyclische Gruppe mit 5, 6 oder 7 Gliedern, gesättigt, teilweise ungesättigt oder aromatisch, möglicherweise umfassend 1, 2 oder 3 Heteroatome, die identisch oder unterschiedlich sind, insbesondere ausgewählt aus Stickstoff, Sauerstoff oder Schwefel, unsubstituiert oder substituiert durch eine oder mehrere, gleichartige oder verschiedene, Substituenten, insbesondere ausgewählt aus:
• eine C₁-C₆-Alkoxygruppe, vorzugsweise C₁-C₃, linear oder verzweigt, zum Beispiel Methoxy, Ethoxy, Propoxy, Iso-Propoxy, Butoxy, Ter-Butoxy;
• ein Halogenatom, zum Beispiel Fluor, Chlor, Brom;
• eine C₁-C₅-Alkylgruppe, vorzugsweise C₁-C₄, linear oder verzweigt, zum Beispiel Methyl, Ethyl, Propyl, Butyl, Isopropyl, Ter-Butyl; oder
• eine C₁-C₅-Alkylgruppe, vorzugsweise C₁-C₄, linear oder verzweigt, insbesondere substituiert durch ein oder mehrere Halogenatome, zum Beispiel Trifluormethyl
▪ R³, R⁴, R⁵ und R⁶, gleich oder verschieden, darstellen:
- ein Wasserstoffatom;
- eine C₁-C₅-Alkylgruppe, linear oder verzweigt, vorzugsweise C₁-C₄.

2. Zusammensetzung nach Anspruch 1, wobei R² darstellt:
- eine 6-gliedrige aromatische Carbocyclus-Gruppe, vorzugsweise nicht am Phenyl substituiert oder substituiert durch einen oder mehrere, gleiche oder verschiedene, Substituenten, insbesondere ausgewählt aus:
• eine C₁-C₆-Alkoxygruppe, vorzugsweise C₁-C₃, linear oder verzweigt, zum Beispiel Methoxy, Ethoxy, Propoxy, Iso-Propoxy, Butoxy, Ter-Butoxy;
• ein Halogenatom, zum Beispiel Fluor, Chlor, Brom;
• eine C₁-C₅-Alkylgruppe, vorzugsweise C₁-C₄, linear oder verzweigt, zum Beispiel Methyl, Ethyl, Propyl, Butyl, Isopropyl, Ter-Butyl;
• eine C₁-C₅-Alkylgruppe, vorzugsweise C₁-C₄, linear oder verzweigt, insbesondere substituiert durch ein oder mehrere Halogenatome, zum Beispiel Trifluormethyl;
- eine 5- oder 6-gliedrige aromatische heterocyclische Gruppe umfassend 1, 2 oder 3 Heteroatome, gleich oder verschieden, insbesondere ausgewählt aus Stickstoff, Schwefel und Sauerstoff, vorzugsweise Pyridin, unsubstituiert oder substituiert durch einen oder mehrere Substituenten, gleich oder verschieden, insbesondere ausgewählt aus:
• eine C₁-C₆-Alkoxygruppe, vorzugsweise C₁-C₃, linear oder verzweigt, zum Beispiel Methoxy, Ethoxy, Propoxy, Iso-Propoxy, Butoxy, Ter-Butoxy;
• ein Halogenatom, zum Beispiel Fluor, Chlor, Brom;
• eine C₁-C₅-Alkylgruppe, vorzugsweise C₁-C₄, linear oder verzweigt, zum Beispiel Methyl, Ethyl, Propyl, Butyl, Isopropyl, Ter-Butyl;
• eine C₁-C₅-Alkylgruppe, vorzugsweise C₁-C₄, linear oder verzweigt, insbesondere substituiert durch ein oder mehrere Halogenatome, zum Beispiel Trifluormethyl.

3. Zusammensetzung nach Anspruch 1 oder 2, wobei R³, R⁴, R⁵ und R⁶, gleich oder verschieden, ein Wasserstoffatom darstellen.

4. Zusammensetzung nach einem der Ansprüche 1 bis 3, wobei R² darstellt:
- eine Phenylgruppe, unsubstituiert oder durch einen oder mehrere Substituenten substituiert, identisch oder verschieden, insbesondere ausgewählt aus:
• eine C₁-C₆-Alkoxygruppe, vorzugsweise C₁-C₃, linear oder verzweigt, zum Beispiel Methoxy, Ethoxy, Propoxy, Iso-Propoxy, Butoxy, Ter-Butoxy;
• ein Halogenatom, zum Beispiel Fluor, Chlor, Brom;
• eine 5- oder 6-gliedrige aromatische heterocyclische Gruppe, umfassend 1, 2 oder 3 Heteroatome, gleich oder verschieden, insbesondere ausgewählt aus Stickstoff, Schwefel und Sauerstoff, vorzugsweise Pyridin.

5. Zusammensetzung nach einem der Ansprüche 1 bis 4, bei der die Verbindung der Formel (I) ausgewählt ist aus:

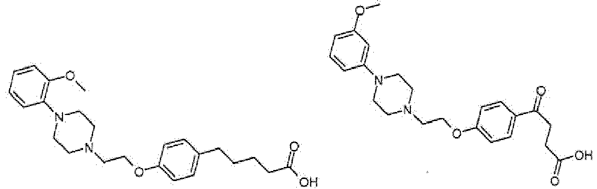

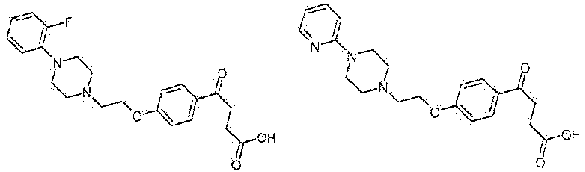

6. Zusammensetzung nach einem der Ansprüche 1 bis 5 zur Verwendung als Medikament.

7. Die Zusammensetzung nach Anspruch 6 zur Verwendung bei der Behandlung von Pathologien im Zusammenhang mit dem Insulinresistenzsyndrom.

8. Zusammensetzung nach Anspruch 7 zur Verwendung bei der Behandlung von Diabetes, insbesondere von Typ-II-Diabetes.

9. Zusammensetzung nach Anspruch 6 zur Verwendung bei der Hemmung der Neoglukogenese.

10. Kit, enthaltend mindestens eine Verbindung der Formel (I) nach den Ansprüchen 1 bis 5, oder ein pharmazeutisch annehmbares Salz davon, und Metformin, oder ein Metforminsalz, für eine gleichzeitige, separate oder sequentielle Gabe an einen Patienten, der Bedarf dafür hat.

## Claims

1. A composition comprising metformin or a salt of metformin, a pharmaceutically acceptable carrier or excipient and at least one formula (I) compound and the enantiomers, diastereoisomers or pharmaceutically acceptable salts thereof: 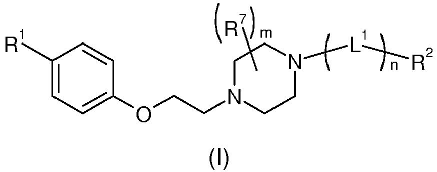 where:
▪ R¹ is a group from among:
- -C(O)CR³R⁴CR⁵R⁶C(O)OH;
- -(CH₂)₄C(O)OH; or
▪ m is 0;
▪ n is 0;
▪ R² is:
- a carbocycle group, 5-, 6 or 7-membered, saturated, partly unsaturated or aromatic, unsubstituted or substituted by one or more substituents identical or different especially chosen among:
• C₁ to C₆ alkoxy group, preferably C₁ to C₃, straight-chain or branched e.g. methoxy, ethoxy, propoxy, iso-propoxy, butoxy, ter-butoxy;
• a halogen atom e.g. fluorine, chlorine, bromine;
• C₁ to C₅ alkyl group, preferably C₁ to C₄, straight-chain or branched e.g. methyl, ethyl, propyl, butyl, iso-propyl, ter-butyl;
• C₁ to C₅ alkyl group, preferably C₁ to C₄, straight-chain or branched, substituted in particular by one or more halogen atoms e.g. trifluoromethyl;
- a heterocycle group, 5-, 6- or 7-membered, saturated, partly unsaturated or aromatic, possibly having 1, 2 or 3 heteroatoms, the same or different, selected in particular from among nitrogen, oxygen or sulphur, unsubstituted or substituted by one or more substituent identical or different, especially chosen among :
• C₁ to C₆ alkoxy group, preferably C₁ to C₃, straight-chain or branched e.g. methoxy, ethoxy, propoxy, iso-propoxy, butoxy, ter-butoxy;
• a halogen atom e.g. fluorine, chlorine, bromine;
• C₁ to C₅ alkyl group, preferably C₁ to C₄, straight-chain or branched e.g. methyl, ethyl, propyl, butyl, iso-propyl, ter-butyl; or
• C₁ to C₅ alkyl group, preferably C₁ to C₄, straight-chain or branched, substituted in particular by one or more halogen atoms e.g. trifluoromethyl;
▪ R³, R⁴, R⁵ and R⁶, the same or different, are:
- a hydrogen atom;
- an alkyl group, straight-chain or branched, C₁ to C₅, preferably C₁ to C₄.

2. The composition according to claim 1 wherein R² is:
- a 6-membered aromatic carbocycle group, preferably phenyl, unsubstituted or substituted by one or more substituents, the same or different, selected in particular from among:
• C₁ to C₆ alkoxy group, preferably C₁ to C₃, straight-chain or branched e.g. methoxy, ethoxy, propoxy, iso-propoxy, butoxy, ter-butoxy;
• a halogen atom e.g. fluorine, chlorine, bromine;
• C₁ to C₅ alkyl group, preferably C₁ to C₄, straight-chain or branched e.g. methyl, ethyl, propyl, butyl, iso-propyl, ter-butyl;
• C₁ to C₅ alkyl group, preferably C₁ to C₄, straight-chain or branched, substituted in particular by one or more halogen atoms e.g. trifluoromethyl;
- 5- or 6-membered heterocycle group having 1, 2 or 3 heteroatoms, the same or different, selected in particular from among nitrogen, sulfur and oxygen, preferably pyridine, unsubstituted or substituted by one or more substituents, the same or different selected in particular from among:
• C₁ to C₆ alkoxy group, preferably C₁ to C₃, straight-chain or branched e.g. methoxy, ethoxy, propoxy, iso-propoxy, butoxy, ter-butoxy;
• a halogen atom e.g. fluorine, chlorine, bromine;
• C₁ to C₅ alkyl group, preferably C₁ to C₄, straight-chain or branched e.g. methyl, ethyl, propyl, butyl, iso-propyl, ter-butyl;
• C₁ to C₅ alkyl group, preferably C₁ to C₄, straight-chain or branched, substituted in particular by one or more halogen atoms e.g. trifluoromethyl.

3. The composition according to claim 1 or 2 wherein R³, R⁴, R⁵ and R⁶ each represent a hydrogen atom.

4. The composition according to anyone of claims 1 to 3, wherein R² is:
- a phenyl group, unsubstituted or substituted by one or more substituents, the same or different, selected in particular from among:
• C₁ to C₆ alkoxy group, preferably C₁ to C₃, straight-chain or branched e.g. methoxy, ethoxy, propoxy, iso-propoxy, butoxy, ter-butoxy;
• a halogen atom e.g. fluorine, chlorine, bromine;
- 5- or 6-membered heterocycle group having 1, 2 or 3 heteroatoms, the same or different, selected in particular from among nitrogen, sulfur and oxygen, preferably pyridine.

5. The composition according to anyone of claims 1 to 4 wherein the formula (I) compound is selected from among:

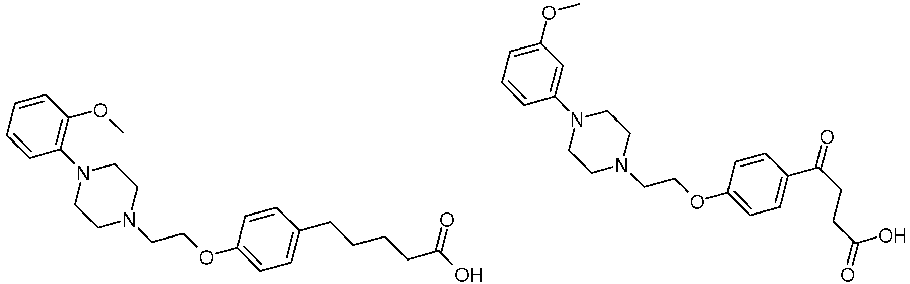

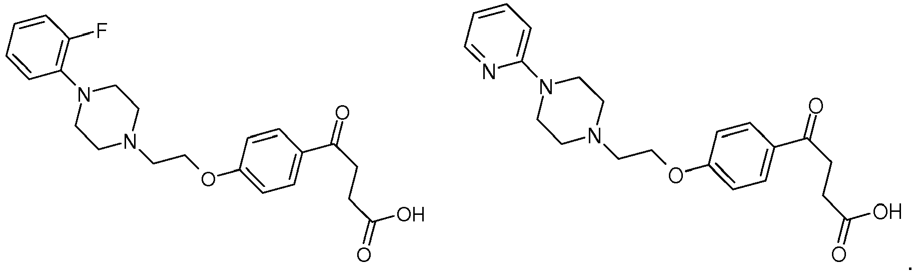

6. Composition according to anyone of claims 1 to 5 for use as a medicine.

7. Composition according to claim 6 for use for the treatment of pathologies associated with insulin-resistance syndrome.

8. Composition according to claim 7 for use for the treatment of diabetes, in particular Type 2 diabetes.

9. Composition according to claim 6 for use for the inhibition of neoglucogenesis.

10. A kit comprising at least one formula (I) compound according to claim 1 to 5 or one of the pharmaceutically acceptable salts thereof, and metformin or a salt of metformin for simultaneous, separate or sequential administration to a patient in need thereof.
